(19) Europäisches Patentamt
European Patent Office
Office européen des brevets

(11) EP 0 938 488 B1

(12) EUROPEAN PATENT SPECIFICATION

(45) Date of publication and mention of the grant of the patent:
**02.01.2002 Bulletin 2002/01**

(21) Application number: **97941092.5**

(22) Date of filing: **22.09.1997**

(51) Int Cl.[7]: **C07F 9/50**, B01J 31/16, B01J 31/24, C07F 9/6539, C07F 15/00, C07C 309/65, C07C 43/20

(86) International application number:
**PCT/GB97/02556**

(87) International publication number:
**WO 98/12202 (26.03.1998 Gazette 1998/12)**

(54) **PHOSPHINE LIGANDS**

PHOSPHIN-LIGANDEN

LIGANDS PHOSPHINIQUES

(84) Designated Contracting States:
**CH DE ES FR GB IT LI NL SE**

(30) Priority: **20.09.1996 GB 9619684**

(43) Date of publication of application:
**01.09.1999 Bulletin 1999/35**

(73) Proprietor: **OXFORD ASYMMETRY INTERNATIONAL PLC**
**Abingdon, Oxon OX14 4SD (GB)**

(72) Inventors:
- **POLYWKA, Mario, Eugenio, Cosamino**
  **Oxon OX11 9PX (GB)**
- **MOSES, Edwin**
  **Berks RG8 9H0 (GB)**
- **BAYSTON, Daniel, John**
  **Bicester OX6 3LE (GB)**
- **BAXTER, Anthony, David**
  **Oxon OX14 5NP (GB)**
- **ASHTON, Mark, Richard**
  **Didcot Oxon OX11 9RW (GB)**

(74) Representative: **Beacham, Annabel Rose et al**
**Frank B. Dehn & Co., European Patent Attorneys,**
**179 Queen Victoria Street**
**London EC4V 4EL (GB)**

(56) References cited:
**EP-A- 0 358 129**
**US-A- 4 178 313**
**US-A- 4 506 030**

- **IVO F. J. VANKELECOM: "Chiral catalytic membranes" ANGEWANDTE CHEMIE. INTERNATIONAL EDITION., vol. 25, no. 12, - 9 July 1996 WEINHEIM DE, pages 1346-1348, XP002047604 cited in the application**
- **SUDO Y ET AL: "Preparation and enantioselectivity of (S)-binaphthol-bonded phase for high-performance liquid chromatography" J. CHROMATOGR., A (JCRAEY,00219673);96; VOL.736 (1 + 2); PP.39-49, CHEMICALS INSPECTION AND TESTING INSTITUTE, DIVISION OF RESEARCH AND DEVELOPMENT, 4-1-1 HIGASHIMUKOJIMA, SUMIDA-KU;TOKYO; 131; JAPAN (JP), XP002047605**
- **BHATT J C ET AL: "Synthesis of highly chiral multisubstituted binaphthyl compounds as potential new biaxial nematic and NLO materials" LIQ. CRYST. (LICRE6,02678292);95; VOL.18 (3); PP.367-80, KENT STATE UNIV.;LIQUID CRYSTAL INST.; KENT; 44242-0001; OH; USA (US), XP000533260**
- **GARCIA-TELLADO F ET AL: "Chiral recognition of tartaric acid derivatives by a synthetic receptor" J. CHEM. SOC., CHEM. COMMUN. (JCCCAT,00224936);91; (24); PP.1761-3, UNIV. PITTSBURGH;DEP. CHEM.; PITTSBURGH; 15260; PA; USA (US), XP002047606**
- **GROVES J T ET AL: "Asymmetric hydroxylation, epoxidation, and sulfoxidation catalyzed by vaulted binaphthyl metalloporphyrins" J. ORG. CHEM. (JOCEAH,00223263);90; VOL.55 (11); PP.3628-34, PRINCETON UNIV.;DEP. CHEM.; PRINCETON; 08544; NJ; USA (US), XP002047607**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- CUNTZE J ET AL: "Molecular clefts derived from 9,9'-spirobi-9H-fluorene for enantioselective complexation of pyranosides and dicarboxylic acids" HELV. CHIM. ACTA (HCACAV,0018019X);95; VOL.78 (2); PP.367-90, EIDGENOESSISCHEN TECH. HOCHSCHULE;LAB. ORG. CHEM.; ZURICH; CH-8092; SWITZ. (CH), XP002047608
- CHEMICAL ABSTRACTS, vol. 126, no. 7, 17 February 1997 Columbus, Ohio, US; abstract no. 089568, KAI D W ET AL: "Method for producing 2,2'-bis(diphenylphosphino)-1,1'-binaphthy l (BINAP) derivatives" XP002047609 & JP 08 311 090 A (MERCK & COMPANY INCORPORATED;USA) 26 November 1996

## Description

This invention relates to new compounds which are suitable for use as ligands for catalysts for use in asymmetric reactions. Such ligands may be attached to an insoluble support in use. This invention also provides a method for the evaluation and use of catalysts comprising such ligands using a combinatorial approach.

Access to enantiomerically pure compounds is essential for the synthesis of natural products, agrochemicals and especially pharmaceuticals. Ideally, asymmetric synthetic techniques are used to produce enantiomerically pure products from prochiral precursors. The most desirable of asymmetric reactions are those employing an asymmetric catalyst. One chiral catalyst molecule can give rise to many chiral product molecules. In addition, catalytic asymmetric synthesis often has significant economical advantages over stoichiometric asymmetric synthesis in the production of enantiomerically pure compounds on an industrial scale. Efforts continue to develop asymmetric reactions with the highest possible stereoselectivity.

A well established class of asymmetric catalysts are transition metal complexes bearing chiral organic ligands. In particular, homogeneous asymmetric catalysis using chiral metal complexes has provided an ideal way to multiply chirality. The appropriate choice of the central metal and the chiral ligand is important for high efficiency of the catalytic process. One important class of catalyst are those based on chiral diphosphines such as 2,2'-bis(diphenylphosphino)-1,1'-binaphthyl (BINAP) (1).

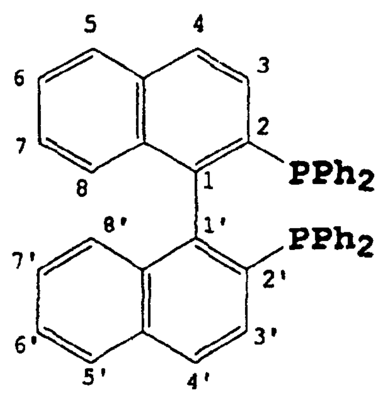

(1)

BINAP has a $C_2$ axis of symmetry and possesses high chemical stability. The free ligand is conformationally flexible enough to accommodate a wide variety of transition metals. Catalysts based on BINAP have shown excellent chiral recognition ability in various asymmetric reactions and BINAP has become one of the more important phosphine ligands for use in producing asymmetric catalysts. (For reviews see Miyashita, A., Takaya, H., Souchi, T., Noyori, R.; *Tetrahedron*, 1245, 40, 1984; European Patent No. 0135392-A2 and European Patent No. 0174057-A2).

In particular the rhodium(I) and ruthenium(II) complexes have been used extensively as chiral catalysts. BINAP coordinated metal complexes have been shown to be efficient catalysts for asymmetric hydrogenation of α-acylaminoacrylic acids and allylic alcohols. They have also been shown to effect an enantioselective 1,3-hydrogen shift of allyl amines to optically active enamines.

Reported examples of highly enantioselective asymmetric reactions utilising BINAP complexes as catalysts include hydrogenation, hydrosilylation, hydroboration of unsaturated compounds, epoxidation of allylic alcohols, vicinal hydroxylation, hydrovinylation, hydroformylation, cyclopropanation, isomerisation of olefins, propylene polymerisation, organometallic addition to aldehydes, allylic alkylation, organic halide-organometallic coupling, aldol type reactions, and Diels-Alder and ene reactions (for reviews see; Noyori, R., *Science* 1990, **248,** 1194; Noyori, R., Kitamura, M., in *Modern Synthetic Methods 1989;* Scheffold, R., Ed., Springer-Verlag: Berlin, 1989; p. 115).

Y. Sudo et al, J. Chromatogr., A 736 (1996), 39-49 discloses a chiral stationary phase for high-performance liquid chromatography wherein (S)-2,2'-dihydroxy-1,1'-binaphthyl is covalently bonded to silica gel. Various 2,2'-dihydroxy-1,1'-binaphthyl derivatives are also disclosed as intermediates in the synthesis of the chiral stationary phase.

EP-A-0358129 discloses various binaphthol derivatives as intermediates in the synthesis of crown ethers.

F. Garcia-Tellado *et al*, J. Chem. Soc., Chem. Commun., 1991, 1761-1763 discloses various binaphthol derivatives as intermediates in the synthesis of a synthetic receptor for the chiral recognition of tartaric acid derivatives.

J.T. Groves *et al*, J. Org. Chem., 1990, 55, 3628-3634, discloses various binaphthol derivatives as intermediates in the synthesis of metalloporphyrin asymmetric catalysts.

J. Cuntze *et al,* Helvetica Chimica Acta, 1995, 78, 367-389 discloses various binaphthol derivatives as intermediates in the synthesis of selective carboxylic acid receptors.

J.C. Bhatt *et al,* Liquid Crystals, 1995, 18(3), 367-380 discloses various binaphthol derivatives as potential nonlinear optical materials.

BINAP is used to prepare some extremely important industrial catalysts used in asymmetric hydrogenations and isomerisations. Catalysts based on BINAP are currently used in the industrial production of (-)-menthol and carbapenems.

However, in the use of a homogeneous, soluble catalyst such as solution phase BINAP, a problem of separation of the catalyst from the reaction products becomes difficult, and usually requires special treatment which destroys the catalyst.. One way to solve this problem would be to fix the catalyst to an insoluble solid support in a way that retains the advantages observed in solution. In fact homogeneous catalysts have been attached to a variety of supports including cross-linked polymers (for a review see Kohler, N., Dawans, F., *Rev. Inst. Fr. Pet.,* 1972, **27,** 105). In this way the catalyst acquires the property of insolubility but may retain the same reactivity exhibited in solution. Once the reaction is completed, the insoluble catalyst may simply be filtered off from the reaction mixture and reused.

BINAP catalysts supported in a thin-film of water or ethylene glycol on the surface of a solid have been reported (Wan, K.T. and Davis, M.E., *Nature*, **370,** 449-450 (1994)).

Recently, there have been reports of BINAP based catalysts which are immobilized by trapping them within elastomeric polydimethylsiloxane membranes (I. F. J. Vankelecom et al., *Angew. Chem. Int. Ed. Engl.,* 1996, **35,** 1346-1347). The catalysts are not physically attached, such as covalently bonded, in any way to the membrane but are simply trapped within the elastomer network. Indeed, it is specifically stated that it is undesirable to attach the catalyst to the membrane as this may interfere with the chirality of the catalyst and hence with its enantioselectivity in asymmetric reactions. The membranes do allow easier separation of the catalysts from the reaction mixtures but there is still the risk of metal leaching into the reactions and contaminating the products.

It is widely accepted that one of the features attributing to the excellent enantioselectivity of BINAP based catalysts is that BINAP itself possesses a $C_2$ axis of symmetry. This is believed to halve the number of possible diastereomeric intermediates involved in the catalytic process and hence to enhance the enantioselectivity.

There is thus still a need for an asymmetric catalyst which is as effective as those based on BINAP itself in a wide range of reactions, but which may be readily attached to an insoluble support to facilitate purification of the reaction products. There is also a need for improved BINAP derivatives in terms of enantioselectivity and processability (ease of separation and purification of the products and reusability of the catalyst). There is also a need for more rapid exploitation of BINAP catalysts, which may be achieved using a combinatorial evaluation approach.

Surprisingly, it has now been found that derivatives of BINAP itself may be attached to an insoluble support and used as ligands for asymmetric catalysts, without any loss of the catalytic activity or enantioselectivity of the catalyst. This is despite the fact that the attachment of BINAP to an insoluble support may break the $C_2$ symmetry that was previously considered essential for the selectivity of BINAP based catalysts.

According to one feature of the invention, there are therefore provided BINAP derivatives of general formula (I) which may be used as ligands for chiral catalysts:

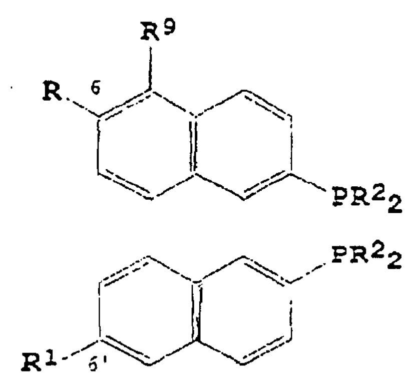

(I)

wherein

R denotes $C_{1-6}$ alkyl, $C_{1-6}$ alkenyl, $C_{1-6}$-alkynyl or phenyl, wherein the $C_{1-6}$ alkyl and phenyl groups may optionally be substituted by one or more substituents which may include F, Cl, Br, $NO_2$, amino, naphthalene, anthracene, biphenyl, $C_{1-5}$ alkyl, $CF_3$, CN, CH, O-$C_{1-6}$ alkyl, $CO_2H$, CHO, NHCO($C_{1-6}$ alkyl), $CO_2$($C_{1-6}$ alkyl), N($C_{1-6}$ alkyl) CO, bensyl, $C_{5-6}$ cyclic ethers or $C_{2-4}$ unsaturated hydrocarbon groups, and wherein the $C_{1-6}$ alkyl group may optionally include one or more intervening heteroatoms or aryl groups in the chain or R denotes CN, $CO_2NHR^3$, $(CH_2)_nOR^3$, $CO_2R^3$, benzyl, heterocyclic groups such as thiophene, furan, pyridine, pyrimidine, quincline, benzofuran, benzothiophene, pyrrole, imidazole, isoquinoline or indole, wherein the heterocyclic groups may be optionally substituted by one or more ether or $C_{1-6}$ alkyl groups, or Y-X-$R^4$;

$R^1$ denotes R or H;

$R^2$ denotes phenyl, phenyl substituted by one or more $C_{1-7}$ alkyl groups, O-$C_{1-6}$ alkyl groups and/or halogen atoms, or $R^2$ denotes a $C_{3-7}$ cyclic aliphatic hydrocarbon group;

$R^9$ denotes H or together with R forms a 5, 6 or 7 membered hydrocarbon ring, optionally substituted by one or more C=O, OH or amine groups;

Y denotes a straight or branched aliphatic chain, optionally incorporating one or mere aromatic hydrocarbon group (s) or ether linkages in the chain, or an aromatic hydrocarbon group;

X denotes $CH_2$, $CO_2$, O, CONH, NH, $CONR^2$, $NR^2$ or a valence bond;

$R^3$ denotes H, $C_1$-$C_{10}$ alkyl, benzyl or phenyl; and

$R^4$ denotes H, $C_1$-$C_6$ alkyl, an insoluble support, or a spacer group attached to an insoluble support;

and all enantiomers, mixtures, including racemic mixtures, and diastereomers thereof.

In the above definitions, alkyl represents a straight or branched alkyl group.

The compounds of general formula (I) may be $C_2$ symmetric or non-symmetric. Preferred compounds are those which consist of a single enantiomer (R) or (S) and are enantiomerically pure.

Preferred compounds of general formula (I) are those wherein

$R^2$ denotes phenyl;

X denotes CONH or $CO_2$;

Y denotes $(CH_2)_n$ wherein n denotes 2 to 4; and

$R^1$ is identical to R.

Further preferred compounds of formula (I) are those wherein

$R^4$ denotes an insoluble support or a spacer group attached to an insoluble support.

Especially preferred are compounds of formula (I) wherein

R2 denotes phenyl;

$R^4$ denotes an insoluble support or a spacer group attached to an insoluble support;

X denotes CONH or $CO_2$; and

Y denotes $(CH_2)_n$ wherein n denotes 2 to 4.

Especially preferred compounds of formula (I) are shown below. Such compounds may exist in the (R) or (S)-enantiomeric form.

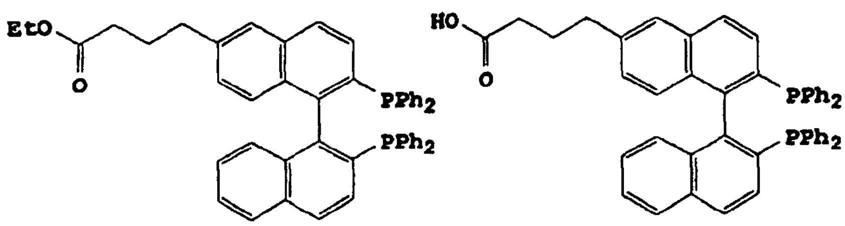

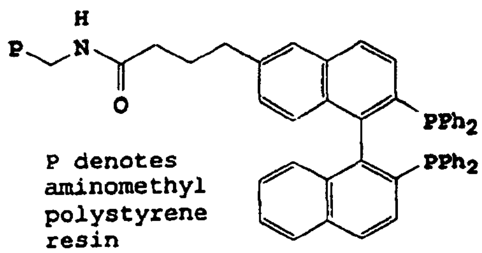

P denotes aminomethyl polystyrene resin

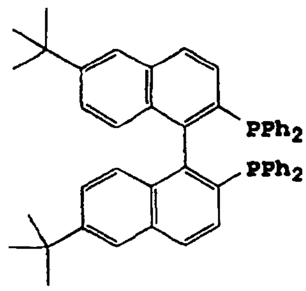

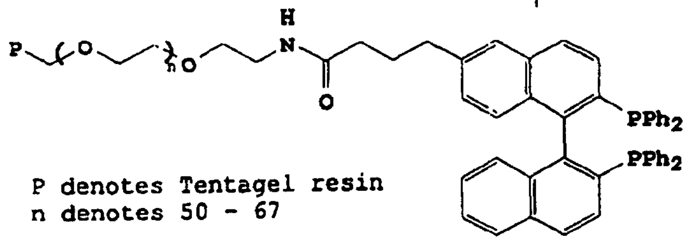

P denotes Tentagel resin
n denotes 50 - 67

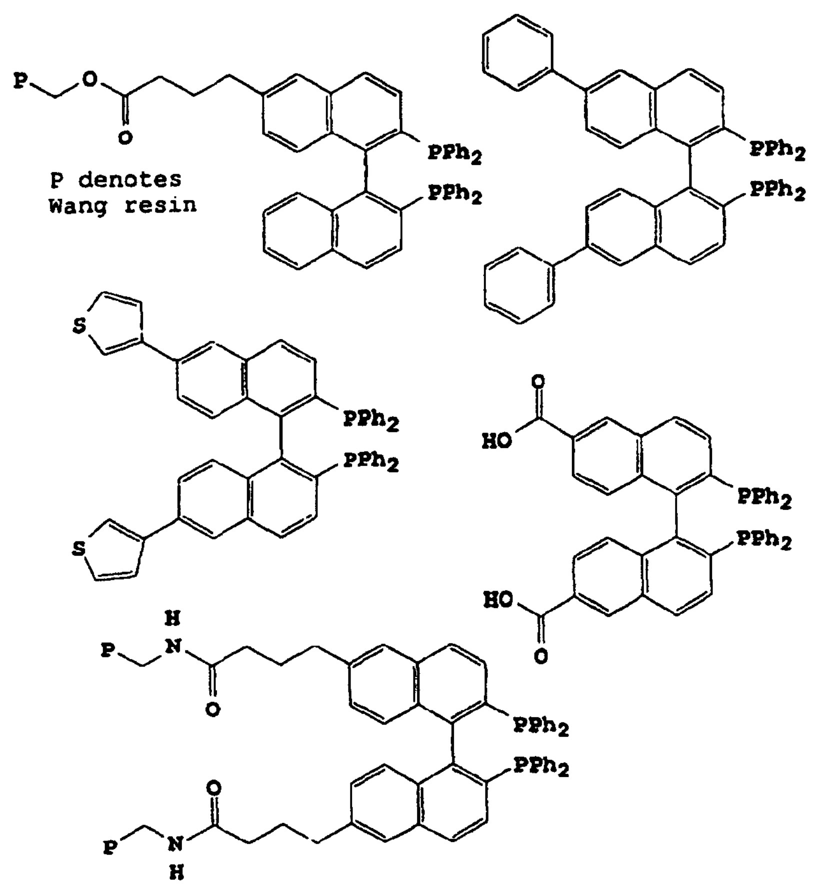

P denotes Wang resin

Examples of insoluble supports include polystyrenedivinyl benzene co-polymer (Merrifield Resin), polystyrene resin, polyamide, aminomethylated polystyrene resin, Wang resin, aminomethylated Tentagel resin, polyamid-kieselguhr composites, polyhipe, cotton, paper and the like.

Preferred insoluble supports are aminomethyl polystyrene resin, Wang resin and Tentagel resin.

The attachment of the X group to the insoluble support may be direct or via a spacer group. Examples of possible spacer groups include alkylene chains and alkylene chains interrupted by ether, amino, ester and/or amide linkages.

The ligands of general formula (I) may be complexed to any transition metal for which BINAP itself is a ligand. Particularly preferred metals for the preparation of asymmetric catalysts are rhodium, ruthenium, and palladium, especially ruthenium.

In a further feature of the invention there are provided complexes comprising a compound of formula (I) complexed to a transition metal.

Such complexes are of use as potential asymmetric catalysts. Such catalysts may be used enantioselectively in solution or when attached to an insoluble support.

In such complexes, the vacant coordination sites on the metal may be occupied by any ligands which complex to the metal in conventional BINAP-based catalysts, or any other ligands which do not affect the catalytic activity of the complex. Such ligands include, but are not limited to, Cl, Br, I, F, allyl, $OCOCH_3$, H, $PCl_6$, $PF_6$, $ClO_4$, $BF_4$, tetraphenylborate, benzene, p-cymene, 1,5-cyclooctadiene, acetylacetonate anion (acac) or tertiary amines such as $NEt_3$.

Possible transition metals include rhodium, ruthenium, palladium, iridium, nickel, cobalt and molybdenum. Particularly preferred for use as asymmetric catalysts are ruthenium, rhodium or palladium complexes.

Preferred for use as asymmetric catalysts are complexes of empirical formula $LRuBr_2$ wherein L denotes a ligand of general formula (I).

Preferred ligands L are compounds of formula (II)

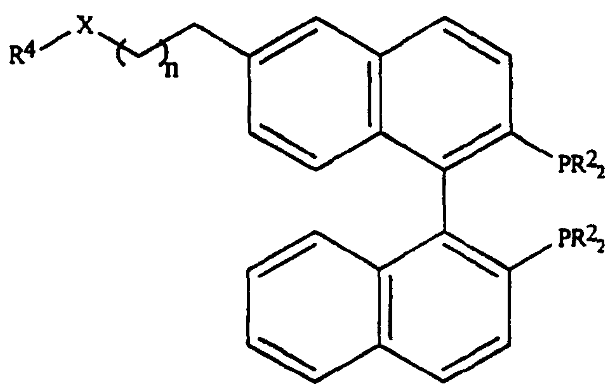

(II)

wherein

| | |
|---|---|
| X | denotes $CO_2$, O, NH, CONH, $CH_2$ or a valence bond; |
| n | denotes 0-9; and |
| $R^2$ and $R^4$ | are as hereinbefore defined. |

The BINAP derivatives of formula (I) may be synthesised in solution and then, if desired, they may be attached to an insoluble solid support using conventional methodology. Attachment to the support may be, for example, by the formation of an amide, ether, amino, ester or carbon-carbon linkage. Scheme 1 illustrates the synthesis of a support-bound compound of formula (I).

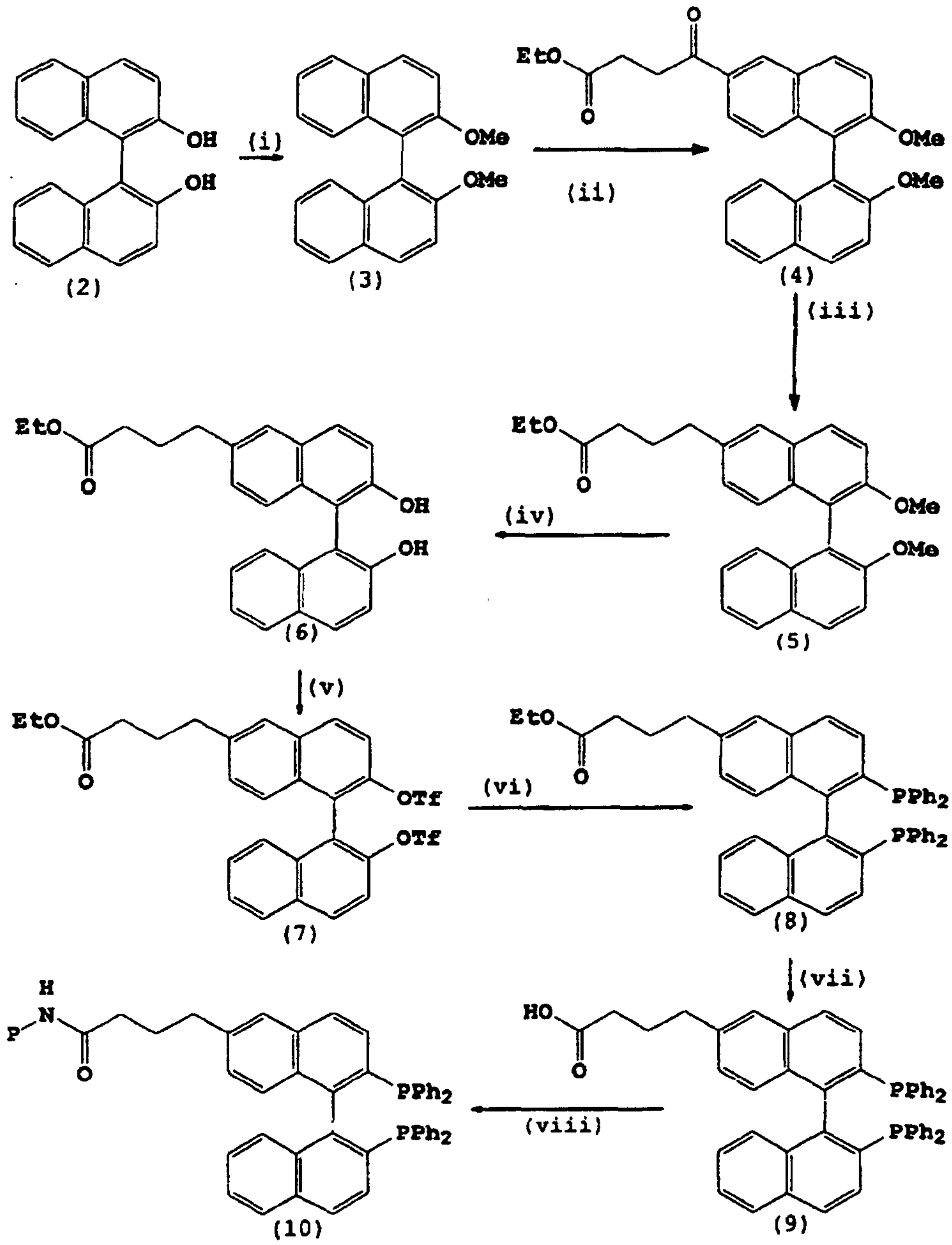

(2) (3) (4) (5) (6) (7) (8) (9) (10)

Scheme 1

(i) MeI, $K_2CO_3$, acetone, reflux; (ii) $EtO_2C(CH_2)_2COCl$, $AlCl_3$, $CH_2Cl_2$; (iii) $H_2$, Pd/C, $CH_3SO_3H$, AcOH, EtOAc, EtOH;
(iv) $BBr_3$, $CH_2Cl_2$; (v) $Tf_2O$, 2,6-lutidine, DMAP, $CH_2Cl_2$;
(vi) $HPPh_2$, $NiCl_2$dppe, DABCO, DMF, 100°C then EtOAc, NaCN then $PhCH_3$, $SiHCl_3$; (vii) LiOH, THF, reflux; (viii) DIC, HOBt, DIPEA, $CH_2Cl_2$, DMF, aminomethylated polystyrene resin (P).

The starting material is enantiomerically pure 1,1'-bi-2-naphthol (2) (BINOL), which is commercially available in either the R-form or the S-form. The synthetic scheme does not affect the chiral integrity of the products. Use of one enantiomer of BINOL as the starting material leads to one enantiomeric product BINAP derivative. Use of the other enantiomer of BINOL as starting material will give the opposite enantiomer of the product.

The alcohol functionalities in BINOL (2) are protected as ethers and a selective Friedel-Crafts acylation is carried out to derivatise one of the naphthyl groups in the 6-position. It was then found necessary to reduce the benzylic carbonyl group in compound (4) to the corresponding methylene group in order to deprotect the methyl ethers. Deprotection of the alcohol groups in compound (4) is followed by a ditriflation. This step is well known in the art for the synthesis of BINAP itself. The phosphine groups are then introduced by displacement of the triflate groups to give compound (8). The side chain may then be further modified and subsequently attached to an insoluble support if required.

Other compounds of the invention may be prepared using analogous procedures to those illustrated in Scheme 1.

As a further feature of the invention there are provided intermediates of formula (III), which may be used in the synthesis of compounds of formula (I).

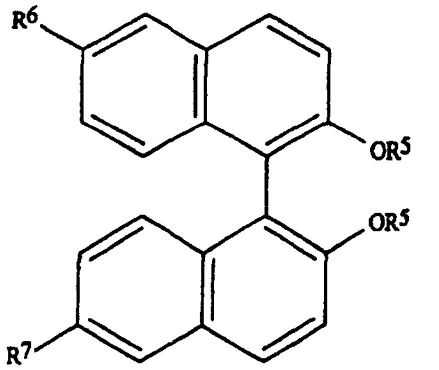

(III)

wherein

$R^5$ denotes any alkyl group which directs substitution to the 6 position, in particular $R^5$ denotes $C_{1-7}$ alkyl, especially $(CH_2)_{0-6}CH_3$, or a $C_{3-7}$ cyclic aliphatic group; and

$R^6$ denotes Cl, a straight or branched acyl or non-acyl aliphatic chain, optionally terminating in an acid functionality and optionally incorporating one or more aromatic hydrocarbon, ether, ester or amide groups within the chain or terminating the chain; or $R^6$ denotes a phenyl group, optionally substituted by one or more F, Cl, Br, $NO_2$, amino, naphthalene, anthracene, biphenyl, $C_{1-6}$ alkyl, $CF_3$, CN, OH, O-$C_{1-6}$ alkyl, $CO_2H$, CHO, NHCO($C_{1-6}$ alkyl), $CO_2$($C_{1-6}$ alkyl), N($C_{1-6}$ alkyl)CO, benzyl, $C_{5-6}$ cyclic ethers or $C_{2-4}$ unsaturated hydrocarbon groups, or $R^6$ denotes a heterocyclic group such as thiophene, furan, pyridine, pyrimidine, quinoline, benzofuran, benzothiophene, pyrrole, imidazole, isoquinoline or indole, wherein the heterocyclic groups may be optionally substituted by one or more ether or $C_{1-6}$ alkyl groups; and

R7 denotes $R^6$ or H;

with the provisos that

a) when $R^5$ denotes methyl and $R^7$ denotes H, then $R^6$ does not denote $-CO(CH_2)_3COOCH_3$, $-CO(CH_2)_3COOH$ or - $(CH_2)_4COOH$; and
b) when $R^7$ denotes $R^6$, then $R^6$ does not denote $C_{1-30}$ alkyl, $C_{6-18}$ aryl or $C_{7-30}$ aralkyl.

Substitution in the 6-position gives a handle via which the BINAP derivatives of the invention may be attached to an insoluble solid support. Derivatisation in the 6-position, or at both the 6 and 6' positions, may be achieved via, for example, Friedel-Crafts acylation, Friedel-Crafts alkylation, Friedel-Crafts arylation cr Suzuki, Heck or Stille coupling reactions on suitable precursors.

Compounds of formula (III) may be synthesised from suitably protected BINOL (2) derivatives of formula (IV). It has now been found that Friedel-Crafts reactions of such compounds proceed highly selectively, depending on the reaction conditions, at either the 6 position or at both the 6 and 6' positions of the naphthyl rings in the compounds of formula (IV).

As a further feature of the invention there is therefore provided a process for the synthesis of compounds of general formula (III) according to Scheme 2.

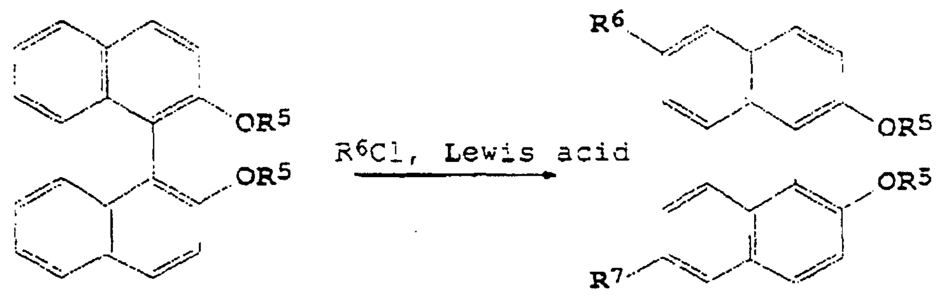


(IV) (III)

Scheme 2

wherein

$R^5$, $R^6$ and $R^7$ are as hereinbefore defined, with the proviso that $R^6$ does not denote Cl.

Suitable Lewis acids include $AlCl_3$, $TiCl_4$, $SnCl_4$, $FeCl_3$, $I_2$, $ZnCl_2$, $BeCl_2$, $CdCl_2$, $BF_3$, $BCl_3$, $BBr_3$, $GaCl_3$, $GaBr_3$, $TiBr_4$, $ZrCl_4$, $SnBr_4$, $SbCl_5$, $SbCl_3$ or $BiCl_3$. A particularly preferred Lewis acid is $AlCl_3$.

Use of a large molar excess of the reagent $R^6Cl$ tends to favour formation of the disubstituted produces ($R^6 = R^7$ in formula (III)). Use of just over one mole equivalent of the reagent $R^6Cl$ tends to favour formation of monosubstituted products ($R^7$ = H in formula (III)).

Alternatively, the 6,6'-disubstituted compounds of the invention may be prepared via elaboration of simple, known 6,6'-disubstituted BINOL derivatives. (R)-6,6'-dibromo-1,1'-bi-2-naphtol may be prepared according to the literature procedure in *J. Am. Chem. Soc.,* 1979, **101,** 3035-3042, while (R) -6,6'-dibromo-2,2'-dimethoxy-1,1'-binaphtyl and (R)-6,6'-dicyano-1,1'-bi-2-naphtol may be prepared according to the literature procedures in *J. Org. Chem.*, 1995, 60, 738B.

For example, the bromo groups in the known compound (R)-6,6'-dibromo-1,1'-bi-2-naphtol may be replaced by aromatic or heterocyclic rings via Suzuki coupling reactions with suitable boronic acid or boronic ester derivatives. For example, treatment of (R) -6,6'-dibromo-1,1'-bi-2-naphtol with thiophene-3-boronic acid may yield (R)-6,6'-di-(3-thienyl)-1,1'-bi-2-naphtol, whilst treatment with phenyl boronic acid may yield the corresponding 6,6'-diphenyl derivative.

The cyanc groups in suitably protected derivatives of the known (R)-6,6'-dicyano-1,1'-bi-2-naphtol may be hydrolysed to yield the corresponding 6,6'-diacid derivatives. The acid groups may then be further elaborated as required using conventional synthetic techniques.

The 6,6'-disubstituted BINOL derivatives may then be converted into che corresponding BINAP compounds of the invention by analogous processes to those mentioned above and illustrated in Scheme 1.

As a further feature of the invention there is provided a process for the preparation of compounds of formula (I) which comprises converting the $OR^5$ groups in a compound of formula (III) into leaving groups which may be displaced by $HPR^2_2$, reacting the resultant product with $HPR^2_2$, wherein $R^2$ is as hereinbefore defined and, if necessary, performing synthetic chemistry to convert the $R^6$ and $R^7$ groups into R and $R^1$ groups respectively.

The introduction of the phosphine groups into the compounds of the invention may be achieved by displacement of a leaving group in a compound of formula (V) by diphenylphosphine or a suitably substituted derivative of diphenylphosphine. As a further feature of the invention there is therefore provided a process for the synthesis of compounds of formula (I) from compounds of formula (V), as shown in Scheme 3.

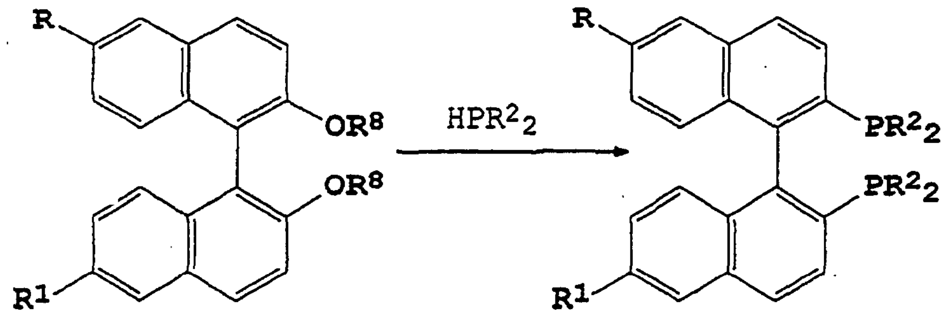

(V)

(I)

Scheme 3

wherein

$OR^8$ denotes a leaving group which may be displaced by $HR^2_2P$, preferably $OSO_2CF_3$ (OTf) ; and
R, $R^1$ and $R^2$ are as hereinbefore defined.

As a further feature of the invention there are provided compounds of formula (V)

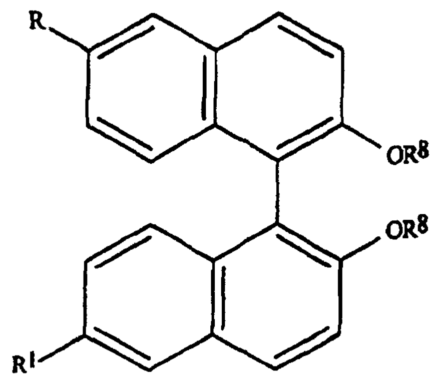

(V)

wherein

$OR^8$ denotes a leaving group which may be displaced by $HPR^2_2$, preferably $OSO_2CF_3$ (OTf); and
R and $R^1$ are as hereinbefore defined.

An alternative method for the introduction of the phosphine groups into the compounds of the invention is via the displacement of bromo groups with suitably substituted derivatives of chlorodiphenylphosphine oxide ($R^2_2POCl$, wherein $R^2$ is as defined above), in an analogous process to that known for the preparation of BINAP itself (Noyori et al., *J. Org. Chem.*, 1986, **51**, 629-635). The resulting phosphine oxide derivatives may then be reduced to the phosphine derivatives by treatment with a reducing agent such as trichlorosilane in the presence of triethylamine.

The BINAP derivatives of general formula (I) may be complexed to transition metals using methods well known in the art for the synthesis of BINAP based catalysts. The complexes of the invention may be produced *in situ*, used without isolation and may be reused after appropriate work-up or regeneration.

Asymmetric catalysts comprising compounds of formula (I) are useful in the same type of reactions as are

conventional BINAP catalysts. In particular, they are of use in the much used asymmetric reductions but their use is not limited to these reactions. As a further feature of the invention there is therefore provided the use of complexes comprising a compound of formula (I) and a transition metal as catalyst in asymmetric reduction reactions, especially in hydrogenations.

Surprisingly, it has been found that the introduction of substituents at the 6-position of BINAP does not affect its ability to act as a chiral ligand in asymmetric catalysts. In particular, it has been found that non-$C_2$ symmetric BINAP catalysts induce the same high enantioselectivity in asymmetric reactions as does the $C_2$ symmetric BINAP molecule itself. Comparison of reactions using catalysts based on BINAP itself and on non-$C_2$ symmetric BINAP derivatives show very similar yields and enantiomeric excesses for both types of catalysts.

The attachment of the BINAP derivatives of the invention to an insoluble support causes, in the majority of cases, little or no reduction in the enantioselectivity of the catalysts compared to the corresponding unsubstituted BINAP catalysts in solution. The attachment may be via only the 6 position of the BINAP derivative of formula (I) or via positions on both naphthyl groups. Such support-bound BINAP derivatives may or may not possess a $C_2$ symmetry axis. The optimal loading of the insoluble support is 0.1-1.0 millimole equivalents per gram, preferably 0.2-0.4 millimole equivalents per gram. The loading of the support may be assessed by weight gain.

The use of such pseudo-heterogeneous catalysts leads to much simpler work-up and purification procedures than does the use of catalysts comprising BINAP itself. The catalyst attached to the solid support is simply filtered off and the resulting filtrate is concentrated to give the desired chiral product with no traces of catalyst or metal complexes contaminating it. Additionally, if the catalyst is filtered off under an inert atmosphere it may be reused without any or only slight reduction in enantioselectivity. The catalyst may be regenerated on the polymer or the polymer bound catalyst may simply be dried and re-used. This is in contrast to homogeneous BINAP catalysts, which may be difficult to remove from the reaction products. In practice, removal of conventional catalysts may entail poisoning the catalyst, therefore removing any hope of re-using it. The potential to re-use the support-bound catalysts of the invention makes them economically attractive compared to conventional BINAP catalysts.

Catalysts comprising the ligands of the invention can be rapidly evaluated using combinatorial chemistry techniques. Combinatorial chemistry (for reviews see Thompson, L.A., Ellman, J.A., *Chem. Rev.* 1996, **96,** 555; Terrett, N.K., Gardner, M., Gordon, D.W., Kobylecki, R.J., Steele, J., *Tetrahedron* 1995, **51,** 8135; Lowe, G., *Chem. Soc. Rev.,* 1995 **309)** is a specialist technique whereby large numbers of molecules may be synthesised with minimal synthetic effort. A key feature of combinatorial chemistry is that compound synthesis is designed such that a range of analogues can be produced using similar reaction conditions, either in the same vessel or individually in parallel using semi-automated synthesis. This has for the most part been used to synthesise large libraries of structurally distinct molecules, such as peptides, for biological evaluation within the pharmaceutical or agrochemical industries

Using specialist apparatus, it is possible to screen a variety of ligands against various substrates under specific reaction conditions. Such apparatus is designed to allow automation of the reactions. For example, for screening catalysts for use in hydrogenation reactions, a high pressure reactor is used which can accommodate a multi vessel reaction block. The use of a solid teflon reaction block in conjunction with a stirrer hot plate, for example an IKALabortechnik RCT basic hot plate stirrer, is preferred. This allows the provision of sufficient heating and mixing of the reaction mixtures, a sufficient supply of hydrogen gas and a suitable amount of sample in each reaction vessel.

The method may be exemplified with reference to the insoluble solid supported bound catalysts of the invention. These support bound catalysts have the advantage that after completion of the reaction, they may simply be filtered off from the products. If the catalyst is filtered off under an inert atmosphere it may be reused or regenerated. The reaction mixtures may be filtered directly into another identical reaction block from which the reaction solvents may be evaporated. The contents of each individual reaction vessel may then be analysed in terms of optical purity, reaction purity, conversion and yield. This allows rapid identification of optimum reaction conditions for a specific reaction and/or substrate. It is anticipated that under optimum reaction conditions it will be possible to achieve optical purities of up to 99% or higher.

This method of screening/evaluating ligands and catalysts may also be used for the solution phase catalysts of the invention, and indeed for the evaluation of other classes of chiral catalysts in solution or when bound to insoluble supports. The following Examples are non-limiting illustrations of the invention.

## Examples

### Example 1

### Preparation of (R)-2,2'-dimethoxy-1,1'-binaphthyl (3)

To a well stirred solution of (R)-BINOL (2) (18.85 g, 0.0659 mol) in anhydrous acetone (600 ml) was added anhydrous $K_2CO_3$ (27.30 g, 0.198 mol) and methyl iodide (28.08 g, 0.198 mol). The mixture was heated at reflux under

a calcium chloride guard tube for 18 hours. After cooling, the volatiles were removed in vacuo and the residual solids dissolved in $CH_2Cl_2$ (600 ml) and $H_2O$ (500 ml). The layers were separated and the aqueous phase extracted with $CH_2Cl_2$ (3 x 200 ml). The combined organic layers were dried over anhydrous $Na_2CO_3$ and the solvent removed in vacuo to leave a pale yellow solid. Purification by washing with MeOH (3 x 50 ml) and drying under reduced pressure yielded (R)-2,2'-dimethoxy-1,1'-binaphthyl (3) as a white solid (18.8 g, 90%). 1H NMR (300MHz, $CDCl_3$): $\delta_H$ 3.80 (6H, s), 7.13 (2H, d, *J* = 8 Hz), 7.23 (2H, dd, *J* = 9, 12 Hz), 7.33 (2H, t, *J* = 9 Hz), 7.48 (2H, d, *J* = 12 Hz), 7.87 (2H, d, *J* = 8 Hz), 8.00 (2H, *d, J* = 9 Hz).

Alternative Preparation of (3)

A mixture of (R)-BINOL (2) (8787 g, 1 mole equivalent), methyl iodide (5 mole eq.) and potassium carbonate (4 mole eq.) in acetone (7.5 vol.) was heated at reflux for 36 hours. The reaction mixture was allowed to cool to room temperature, and the solids removed by filtration. The residual solids were then washed with water (3 x 5 vol.), to remove inorganic material, and ethyl acetate (1 x 4 vol.), and dried in a vacuum oven at 40°C overnight. The desired product was obtained as a white solid in 94% yield (9046 g). (1 Volume of solvent corresponds to 1 litre per kg of substrate).

Example 2

Preparation of (R)-acylated dimethoxyBINOL (4)

To a cooled (0°C) solution of (R)-2,2'-dimethoxy-1,1'-binaphthyl (3) (8.46 g, 0.027 mol) in $CH_2Cl_2$ (200 ml) under argon was added solid $AlCl_3$ (3.94 g, 0.030 mol). The red solution was stirred for 10 minutes and to this was added dropwise ethyl succinyl chloride (4.88 g, 0.030 mol). The resulting brown solution was warmed to room temperature, stirred for 18 hours and then poured carefully onto $H_2O$ (200 ml). The layers were separated and the aqueous phase extracted with $CH_2Cl_2$ (2 x 100 ml). The combined organic fractions were dried over anhydrous $Na_2SO_4$ and the solvents removed in vacuo. Purification was affected by flash column chromatography (silica gel, EtOAc-hexane, 30%) to yield the title product (R)-(4) as a white solid (7.15 g, 60 %).
1H NMR (300MHz, $CDCl_3$): $\delta_H$ 1.28 (3H, t, *J* = 7 Hz), 2.80 (2H, t, *J* = 8 Hz), 3.41 (2H, t, *J* = 8 Hz), 3.75 (3H, s), 3.79 (3H, s), 4.18 (2H, q, *J* = 7 Hz), 7.10 (1H, d, *J* = 9 Hz), 7.20 (1H, d, *J* = 9 Hz), 7.24 (1H, t, *J* = 9 Hz), 7.33 (1H, t, *J* = 9Hz), 7.48 (1H, d, *J* = 9 Hz), 7.52 (1H, d, *J* = 9 Hz), 7.80 (1H, d, *J* = 9 Hz), 7.89 (1H, d, *J* = 9 Hz), 8.00 (1H, d, *J* = 9 Hz), 8.12 (1H, d, *J* = 9 Hz), 8.57 (1H, s).

Example 3

Preparation of (R)-hydrogenated dimethoxyBINOL (5)

A flask containing (R)-(4) (5.44 g, 0.0123 mol), 10% Pd on carbon (0.75 g), methanesulphonic acid (1.42 g, 0.0148 mol), acetic acid (2.5 ml), EtOAc (85 ml) and EtOH (85 ml) was thoroughly purged with argon and then hydrogen. The reaction mixture was stirred under an atmosphere hydrogen for 18 hours, filtered through celite and the solvents removed in vacuo. The residue was dissolved in EtOAc (100 ml) and treated with saturated aqueous $NaHCO_3$ (100 ml). The phases were separated and the aqueous layer extracted with EtOAc (3 x 30 ml). The combined organic extracts were dried ($Na_2CO_3$) and the volatiles removed in vacuo. Purification by flash column chromatography (silica gel, EtOAc-hexane, 15%) yielded the title product (R)-(5) as a clear oil which solidified upon standing (4.20 g, 80%).
1H NMR (300MHZ; $CDCl_3$) : $\delta_H$ 1.22 (3H, t, *J* = 7 Hz), 1.95-2.08 (2H, m), 2.31 (2H, t, *J* = 8 Hz), 2.72 (2H, t, *J* = 8 Hz), 3.74 (3H, s), 3.78 (3H, s), 4.12 (2H, q, *J* = 7Hz), 7.01-7.08 (2H, m), 7.09 (1H, d, *J* = 9Hz), 7.17-7.27 (1H, m), 7.29 (1H, t, *J* = 9 Hz), 7.40-7.47 (2H, m), 7.61 (1H, s), 7.84 (1H, d, *J* = 9 Hz), 7.92 (1H, d, *J* = 9 Hz), 7.98 (1H, d, *J* = 9 Hz).

Example 4

Preparation of (R)-demethylated material (6)

To a cooled (-78°C) solution of (R)-(5) (0.99 g, 2.31 mmol) in anhydrous $CH_2Cl_2$ (15 ml) was added dropwise a 1.0 M $CH_2Cl_2$ solution of $BBr_3$ (5.1 ml, 5.10 mmol). The mixture was warmed slowly to room temperature, stirred for 1.5 hours and poured carefully onto saturated aqueous $NaHCO_3$ (50 ml). The layers were separated and the aqueous phase extracted with $CH_2Cl_2$ (3 x 20 ml). The combined organic layers were dried over anhydrous $Na_2CO_3$ and the solvent removed in vacuo. Flash column chromatography (silica gel, EtOAc-hexane, 20 %) provided the title product (R)-(6) as a white solid (0.69 g, 75 %).

1H NMR (300MHz, $CDCl_3$) : $\delta_H$ 1.23 (3H, t, *J* = 7 Hz), 1.98-2.03 (2H, m), 2.32 (2H, t, *J* = 8 Hz), 2.75 (2H, t, *J* = 8 Hz), 4.11 (2H, q, *J* = 7 Hz), 5.02 (1H, s), 5.10 (1H, s), 7.08 (1H, d, *J* = 9 Hz), 7.16 (2H, d, *J* = 9 Hz), 7.27-7.42 (4H, m), 7.58 (1H, s), 7.85-7.90 (2H, m), 7.97 (1H, d, *J* = 9 Hz).

Example 5

Preparation of (R)-ditriflate (7)

To a cooled (0°C) mixture of (R)-(6) (0.67 g, 1.68 mmol), 2,6-lutidine (0.45 g, 4.19 mmol) and 4-dimethylaminopyridine (0.020 g, 0.169 mmol) was added dropwise trifluoromethanesulphonic anhydride (1.04 g, 3.69 mmol). The resulting orange solution was warmed to room temperature, stirred for 20 hours and then poured onto saturated aqueous $NaHCO_3$ (20 ml). The layers were separated and the aqueous phase extracted with $CH_2Cl_2$ (3 x 15 ml). The combined organic layers were washed with 0.5 M aqueous HCl (20 ml) and $H_2O$ (20 ml) then dried over anhydrous $Na_2CO_3$. Removal of the solvent in vacuo and purification by flash column chromatography (silica gel, EtOAc-hexane, 15%) gave the title product (R)-(7) as a colourless oil (0.92 g, 83 %).
1H NMR (300MHz, $CDCl_3$) : $\delta_H$ 1.26 (3H, t, *J* = 7 Hz), 2.02-2.10 (2H, m), 2.37 (2H, t, *J* = 8 Hz), 2.82 (2H, t, *J* = 8 Hz), 4.13 (2H, q, *J* = 7 Hz), 7.18 (1H, d, *J* = 9 Hz), 7.24-7.29 (2H, m), 7.41 (1H, t, *J* = 9 Hz), 7.58-7.66 (3H, m), 7.79 (1H, s), 8.01 (1H, d, *J* = 9 Hz), 8.07 (1H, d, *J* = 9 Hz), 8.14 (1H, d, J = 9 Hz).

Example 6

Preparation of (R)-diphosphine (8)

A solution of $NiCl_2$dppe (2.12 g, 4.01 mmol) in anhydrous DMF (10 ml) was degassed thoroughly using 7 pump/argon cycles. $HPPh_2$ (1.24 g, 6.68 mmol) was added and the red mixture aged at 100°C for 1 hr. In a separate flask were degassed (R)-(7) (2.22 g, 3.34 mmol) and 1,4-diazabicyclo[2.2.2]octane (1.50 g, 0.0134 mol) in DMF (10 ml) and added to the nickel solution via canula. The resulting deep green solution was heated at 100°C, a further portion of $HPPh_2$ (1.24 g, 6.68 mmol) added after 4 hours and continued heating for a further 16 hours. After cooling to room temperature the mixture was diluted with EtOAc (50 ml), poured onto 50 ml aqueous NaCN (1.64 g, 0.0334 mmol) and stirred vigorously for 1 hr. The layers were separated and the organic phase washed with $H_2O$ (3 x 20 ml), dried over anhydrous $Na_2SO_4$ and the solvents removed in vacuo. The resulting brown solid was then dissolved in anhydrous toluene (50 ml), treated with trichlorosilane (13.42 g, 0.099 mol) and heated at reflux for 18 hours. The mixture was quenched by pouring carefully onto 2.0 M aqueous NaOH (100 ml) and stirring vigorously for 30 mins. The layers were separated and the aqueous phase extracted with $CH_2Cl_2$ (3 x 20 ml). The combined organic layers were dried over anhydrous $Na_2CO_3$ and the volatiles removed in vacuo. Purification was affected by flash column chromatography (silica gel, EtOAc-hexane, 10 %) to give the title product (R)-(8) as a white solid (1.64 g, 66 %).
1H NMR (300MHz, $CDCl_3$) : $\delta_H$ 1.30 (3H, t, *J* = 7 Hz), 1.94-2.03 (2H, m), 2.31 (2H, t, *J* = 8 Hz), 2.72 (2H, t, *J* = 8 Hz), 4.16 (2H, q, *J* = 7 Hz), 6.71 (2H, s), 6.89 (1H, d, *J* = 9 Hz), 6.95 (1H, t, *J* = 9 Hz), 7.06-7.25 (10H, m), 7.37 (1H, t, *J* = 9 Hz), 7.48 (2H, d, *J* = 9 Hz), 7.62 (1H, s), 7.83-7.88 (2H, m), 7.92 (1H, d, *J* = 9 Hz).

Example 7

Preparation of (R) - acid (9)

To a solution of (R)-(8) (1.48 g, 2.01 mmol) in THF (15 ml) was added 15 ml aqueous LiOH (4.0 g, 0.10 mol) and the mixture heated at reflux for 20 hours. After cooling to room temperature the solution was acidified to pH 3 with 2.0 M aqueous HCl and extracted with EtOAc (3 x 20 ml). The combined organic extracts were dried over anhydrous $Na_2CO_3$ and the solvent removed in vacuo. Recrystallisation from methanol afforded the title compound (R)-(9) as a white solid (1.43 g, 99 %).

Example 8

Aminomethyl polystyrene supported BINAP (10)

Aminomethyl polystyrene resin (1.0 g, 0.21 mmol) was swollen with $CH_2Cl_2$ (5 ml). (R)-9 (0.223 g, 0.315 mmol) was added as a solution in DMF (5 ml) followed by hydroxybenzotriazole (0.064 g, 0.42 mmol), diisopropylethylamine (0.030 g, 0.21 mmol) and diisopropylcarbodiimide (0.056 g, 0.44 mmol). The resulting mixture was stirred slowly for 24 hr. The resin was collected by filtration and washed sequentially with DMF (2 x 5 ml), $CH_2Cl_2$ (2 x 5 ml), MeOH

(2 x 5 ml) and $Et_2O$ (2 x 5 ml). Drying under vacuum afforded a white coloured resin (R)-10 (1.148 g, quantitative loading) to be used in asymmetric hydrogenation reactions.

Example 9

Asymmetric Hydrogenations, Typical Catalyst Preparation:

To a mixture of (R)-diphosphine-resin (10) (30 mg, 0.0063 mmol) and bis-(2-methylallyl)cycloocta-1,5-dieneruthenium (II) complex (2 mg, 0.0063 mmol) in anhydrous degassed acetone (0.5 ml) was added 0.29 M methanolic HBr (0.043 ml, 0.0125 mmol). The amber mixture was stirred at room temperature for 1 hr and the solvent removed thoroughly in vacuo to leave the coloured active resin which was used immediately as a hydrogenation catalyst.

Example 10

Typical hydrogenation procedure:

A solution of methyl propionylacetate (41 mg, 0.314 mmol) in degassed THF (0.3 ml) and MeOH (0.3 ml) was added to catalyst in a glass vial and placed into a stainless steel pressure vessel. The system was thoroughly purged with hydrogen by three cycles of pressurising and stirred magnetically with heating at 50°C under 10 atmospheres of hydrogen pressure for 18 hr. After cooling the reaction mixture was filtered and the resin washed with THF (3 x 1 ml). Removal of solvent in vacuo furnished the β-hydroxy ester which was analysed without purification. Enantiomeric excess = 96.9%.
1H NMR (300MHz, $CDCl_3$) : $\delta_H$ 0.95 (3H, t, $J$ = 6 Hz), 1.43-1.60 (2H, m) , 2.42 (1H, dd, $J$ = 9, 12 Hz), 2.53 (1H, dd, 4, 12 Hz), 2.96 (1H, s), 3.72 (3H, s), 3.90-4.00 (1H, m).

Example 11

(R) -6,6'-Di-t-butyl-2,2'-dimethoxy-1,1'-binaphthyl

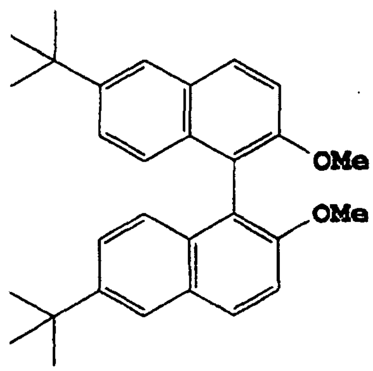

t-Butyl chloride (3.0 g, 31.8 mM) was added to a stirred solution of (R)-2,2'-dimethoxy-1,1'-binaphthyl (3) (1.0 g, 3.18 mM) in dichloromethane (30 ml) at -78°C under an atmosphere of argon. To the mixture was added aluminium chloride (4.24 g, 31.8 mM) and the mixture stirred for a further 6 hours at -78°C. The reaction mixture was allowed to warm to room temperature and quenched by the dropwise addition of water (50 ml). Dichloromethane (2x30 ml) was added and the organic layers were separated, dried over magnesium sulphate, filtered and the solvent removed *in vacuo.* Purification by column chromatography eluting with 30% ethyl acetate:hexane gave the title compound (1.1 g, 81%) as a white solid.

Example 12

(R)-6,6'-Di-t-butyl-1,1'-bi-2-naphthol

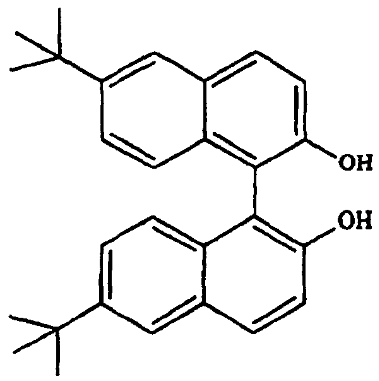

To a pre-cooled (-78°C) stirred solution of (R)-6,6'-di-t-butyl-2,2'-dimethoxy-1,1'-binaphthyl (1.08g, 2.92 mM), prepared according to Example 11, in dichloromethane (10 ml) under an atmosphere of argon was added dropwise boron tribromide (0.6 ml, 5.82 mM). The resulting black solution was allowed to warm to room temperature and stirred for 2 hours. The reaction mixture was quenched by the dropwise addition of water (10 ml), and the resulting phases separated. The aqueous phase was washed with dichloromethane (2 x 20 ml) and the combined organic extracts dried over sodium sulphate and concentrated *in vacuo*. Purification by column chromatography eluting with 20% ethyl acetate: hexane gave the title compound as a colourless oil (0.91g, 91%).

Example 13

(R)-6,6'-di-t-butyl-2,2'-ditrifluoromethansulphonate-1,1'-binaphthyl

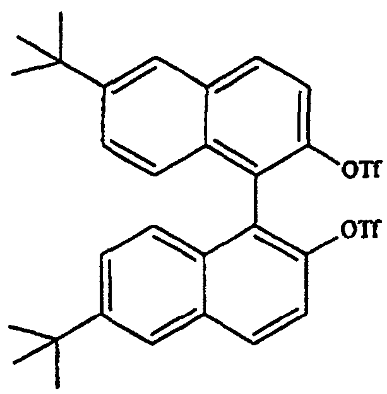

Trifluoromethanesulphonic anhydride (0.6 ml, 3.6 mM) was added to a stirred solution of (R)-6,6'-di-t-butyl-1,1'-bi-2-naphthol (1.19g, 3.5 mM), prepared according to Example 12, 4-dimethylaminopyridine (6 mg) and 2,6-lutidine (1.0 ml) in dichloromethane (10 ml) at 0°C under an atmosphere of argon. The reaction mixture was allowed to warm to room temperature and stirred for a further 16 hours. Saturated sodium bicarbonate solution (10 ml) was added to the reaction mixture to quench and the organic layer separated. The aqueous phase was washed successively with dichloromethane (2 x 15 ml). The combined organic extracts were dried over sodium sulphate and the solvent removed *in vacuo* to give an oil. Purification by column chromatography eluting with 1% ethyl acetate:hexane gave the title compound as a colourless oil (1.63g, 85%).

Example 14

(R)-di-t-butyl-2,2'-bis(diphenylphosphino)-1,1'-naphthyl

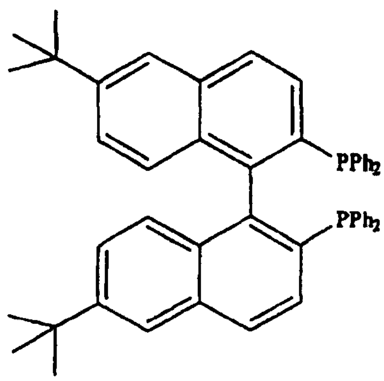

A solution of $NiCl_2$dppe (1.13g, 2.14 mM) in anhydrous dimethylformamide (10 ml) was degassed thoroughly using 7 pump/argon cycles. Diphenylphosphine (0.62 ml, 1.9 mM) was added and the red mixture aged at 100°C for 1 hour. In a separate flask were degassed (R)-6,6'-di-t-butyl-2,2'-di-trifluoromethanesulphonate-1,1'-binaphthyl (1.0g, 1.65 mM), prepared according to Example 13, and 1,4-diazabicyclo[2.2.2]octane (0.81g, 7.2 mM) in dimethylformamide (10 ml) and added to the nickel solution via canula. The resulting deep green solution was heated at 100°C, a further portion of diphenylphosphine (0.62 ml, 1.9 mM) added after 4 hours and continued heating for a further 16 hours. After cooling to room temperature the mixture was diluted with ethyl acetate (30 ml), poured onto 50 ml aqueous sodium cyanide (0.86g, 17.9 mM) and stirred vigorously for 1 hour. The layers were separated and the organic phase washed with water (3 x 20 ml), dried over anhydrous sodium sulphate and the solvents removed *in vacuo.* The resulting brown solid was then dissolved in anhydrous toluene (30 ml), treated with trichlorosilane (0.97g, 0.73 mM) and heated at reflux for 18 hours. The mixture was quenched by pouring carefully onto 2.0 M aqueous sodium hydroxide (30 ml) and stirring vigorously for 30 minutes. The layers were separated and the aqueous phase extracted with Dichloromethane (3 x 25 ml). The combined organic layers were dried over anhydrous sodium carbonate and the solvent removed *in vacuo.*
Purification was effected by flash column chromatography (silica gel, ethyl acetate-hexane, 10%) to give the title compound as a white solid (0.75g, 62%).

Example 15

(R)-6,6'-Dibromo-2,2'-dibenzoxy-1,1'-binaphthyl

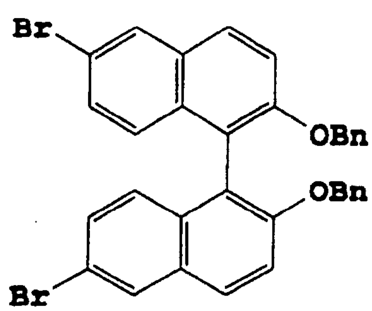

(R)-6,6'-Dibromo-1,1'-bi-2-naphthol (0.50 g, 1.13 mM), prepared according to the literature procedure in *J. Am. Chem. Soc.* 1979, **101,** 3035-3042, benzyl bromide (0.40 ml, 3.38 mM) and potassium carbonate (0.78 g, 5.65 mM) were stirred in refluxing acetone (10 ml) under an atmosphere of argon for 18 hours. After cooling to room temperature, the reaction mixture was poured into dichloromethane (25 ml) and water (25 ml). The layers were separated and the aqueous layer extracted with dichloromethane (2 x 25 ml). The combined organic layers were dried over sodium sulphate, filtered and the solvent removed *in vacuo*. The residue was purified by trituration with hexane and the title compound isolated as a white solid (0.58 g, 83%).

Example 16

(R)-6,6'-Dicyano-2,2'-dibenzoxy-1,1'-binaphthyl

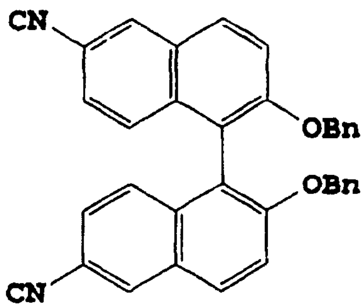

A solution of (R)-6,6'-dibromo-2,2'-dibenzoxy-1,1'-binaphthyl (5.60 g, 8.97 mM), prepared according to Example 15, copper (I) cyanide (3.23 g, 36.0 mM) in DMF (50 ml) was stirred at 170°C for 12 hours under an atmosphere *of* argon. After cooling the reaction mixture was poured onto aqueous sodium cyanide (100 ml) and the resulting mixture stirred until all the dark solids were quenched to give a pale brown slurry. The solids were collected by filtration, dissolved in dichloromethane, dried over sodium sulphate and the solvent removed in *vacuo* to give a brown solid. Purification by column chromatography eluting with 30% ethyl acetate:hexane gave the title compound as a white solid (3.27 g, 71%).

Example 17

(R)-6,6'-Dicarboxy-2,2'-dibenzoxy-1,1'-binaphthyl

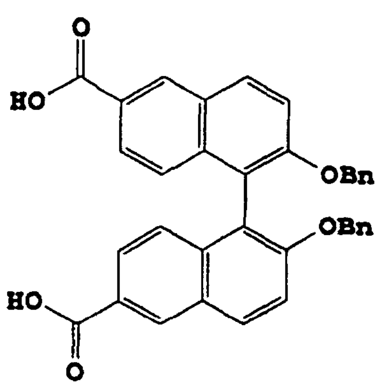

2N sodium hydroxide (100 ml) was added to a stirred solution of (R)-6,6'-dicyano-2,2'-dibenzoxy-1,1'-binaphthyl (3.27 g, 6.34 mM), prepared according to Example 16, in 2-methoxy ethanol (50 ml). The resulting reaction mixture

was stirred at reflux for 24 hours after which it was cooled to room temperature and acidified with 2N HCl to pH 4. The resulting white precipitate was collected by filtration and washed with water. The white solid was then washed with acetone and the filtrate concentrated *in vacuo* to give the title compound as a white solid (2.35 g, 67%).

Example 18

(R)-6,6'-di-methylcarboxy-2,2'-dibenzoxy-1,1'-binaphthyl

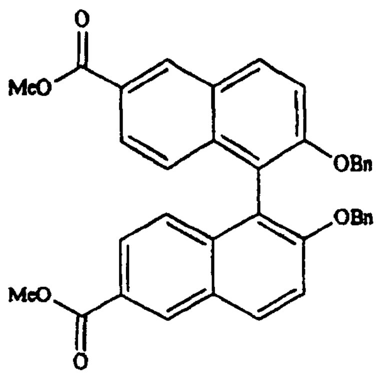

A stirred solution of (R)-6,6'-dicarboxy-2,2'-dibenzoxy-1,1'-binaphthyl (2.35g, 4.24mM), prepared according to Example 17, methyl iodide (1.1ml, 17.0mM) and potassium carbonate (2.93g, 21.2mM) in anhydrous acetone (50ml) was refluxed under argon for 16 hours. After cooling to room temperature the solvent was removed *in vacuo.* The residue was dissolved in dichloromethane (50ml) and water (50ml). The layers were separated and the aqueous layer washed with dichloromethane (2 x 50ml). The combined organic layers were dried over sodium sulphate and concentrated *in vacuo* to give a pale yellow oil. Purification by column chromatography eluting with 10% ethyl acetate:hexane yielded the title compound as an off-white solid (2.39g, 97%).

Example 19

(R)-6.6'-di-methylcarboxy-1,1'-bi-2-naphthol

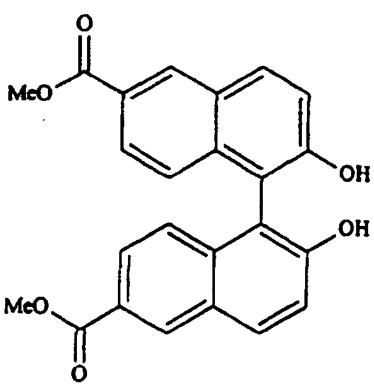

10% Palladium on carbon (1.0g) was added under an atmosphere of argon to a degassed stirred solution of (R)-6,6'-di-methylcarboxy-2,2'-dibenzoxy-1,1'-binaphthyl (2.35g, 4.04mM), prepared according to Example 18, in ethyl acetate (40ml) and methanol (40ml). The resulting suspension was stirred under an atmosphere of hydrogen at atmospheric pressure for 16 hours. The reaction mixture was filtered through a pad of celite and the solid washed with ethyl acetate (3 x 20ml). The solvent was removed *in vacuo* to give a white solid (1.62g, 99%) which required no further

purification.

Example 20

(R)-6,6'-di-methylcarboxy-2,2'-ditrifluoromethanesulphonate-1,1'-binaphthyl

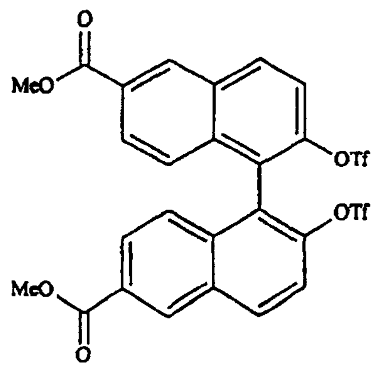

Trifluoromethanesulphonic anhydride (70mg, 0.25mM) was added to a stirred solution of (R)-6,6'-dimethylcarboxy-1,1'-bi-2-naphthol (40mg, 0.095 mM), prepared according to Example 19, 4-dimethylaminopyridine (5mg) and 2,6-lutidine (28mg, 0.26mM) in dichloromethane (5ml) at 0°C under an atmosphere of argon. The reaction mixture was allowed to warm to room temperature and stirred for a further 16 hours. Saturated sodium bicarbonate solution (10ml) was added to the reaction mixture to quench and the organic layer separated. The aqueous phase was washed successively with dichloromethane (2 x 10ml). The combined organic extracts were dried over sodium sulphate and the solvent removed *in vacuo* to give an oil. Purification by column chromatography eluting with 30% ethyl acetate:hexane gave the title compound as a pale brown solid (60mg, 91%).

Example 21

(R) -6,6'-di-methylcarboxy-2,2'-bis(diphenylphosphino)-1,1'-binaphthyl

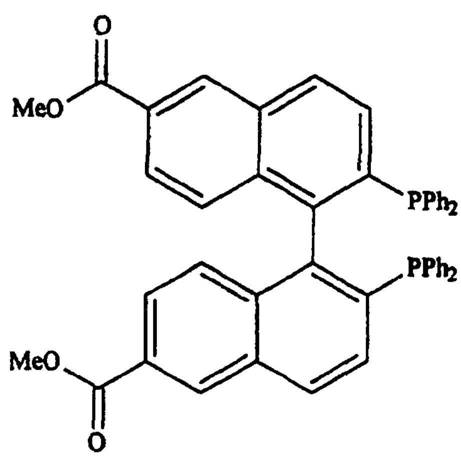

A solution of $NiCl_2$dppe (95mg, 0.18mM) in anhydrous dimethylformamide (1 ml) was degassed thoroughly using 7 pump/argon cycles. Diphenylphosphine (56mg, 0.15ml) was added and the red mixture aged at 100°C for 1 hour. In a separate flask were degassed (R)-6,6'-dimethylcarboxy-2,2'-di-trifluoromethanesulphonate-1,1'-binaphthyl (100mg, 0.15mM), prepared according to Example 20, and 1,4-diazabicyclo[2.2.2]octane (67mg, 0.60mM) in dimethylformamide (1 ml) and added to the nickel solution via canula. The resulting deep green solution was heated at 100°C, a further portion of diphenylphosphine (56mg, 0.15ml) added after 4 hours and continued heating for a further 16 hours. After cooling to room temperature the mixture was diluted with ethyl acetate (5 ml), poured onto 50 ml aqueous sodium cyanide (74mg, 1.5mM) and stirred vigorously for 1 hour. The layers were separated and the organic phase washed with water (3 x 5 ml), dried over anhydrous sodium carbonate and the solvents removed *in vacuo.* The resulting brown solid was then dissolved in anhydrous toluene (5 ml), treated with trichlorosilane (0.5ml) and heated at reflux for 18 hr. The mixture was quenched by pouring carefully onto 2.0 M aqueous sodium hydroxide (5 ml) and stirring vigorously for 30 mins. The layers were separated and the aqueous phase extracted with dichloromethane(3 x 5 ml). The combined organic layers were dried over anhydrous sodium carbonate and the solvent removed *in vacuo*. Purification was affected by flash column chromatography (silica gel, ethyl acetatehexane, 20 %) to give the title compound as an off-white solid (67mg, 58%).

Example 22

(R)-6,6'-di-carboxy-2,2'-bis(diphenylphosphino) -1,1'-binaphthyl

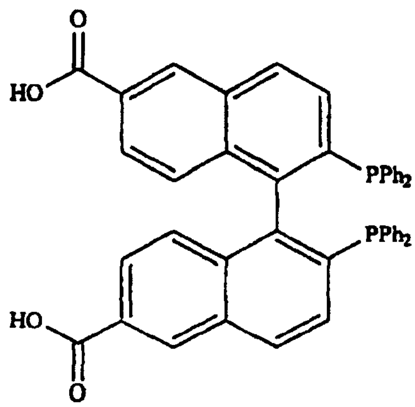

To a solution of (R)-6,6'-di-methylcarboxy-2,2'-bis(diphenylphosphino)-1,1'-binaphthyl (0.57g, 0.74mM), prepared according to Example 21, in tetrahydrofuran (5 ml) was added 15 ml aqueous lithium hydroxide (0.1 g, 4.2mM) and the mixture heated at reflux for 20 hr. After cooling to room temperature the solution was acidified to pH 3 with 2.0 M aqueous HCl and extracted with ethyl acetate (3 x 10 ml). The combined organic extracts were dried over anhydrous sodium carbonate and the solvent removed *in vacuo.* Recrystallisation from methanol afforded the title compound as a white solid (0.53g, 97 %).

Example 23

(R) -6,6'-Di-(3-thienyl)-1,1'-bi-2-naphthol

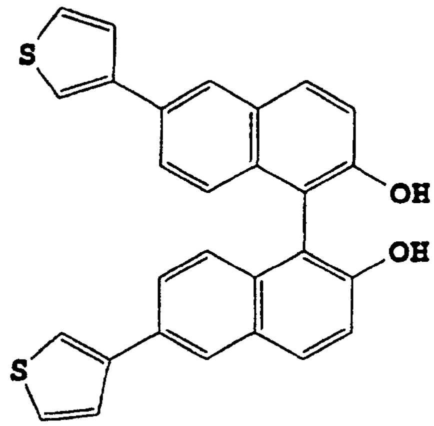

A solution of (R)-6,6'-dibromo-1,1'-bi-2-naphthol (4.0 g, 9.01 mM), prepared according to the literature procedure in *J. Am. Chem. Soc.* 1979, **101**, 3035-3042, and tetrakis(triphenylphosphine)palladium (O) (476 mg, 0.41 mM) in ethylene glycol dimethyl ether (DME) (40 ml) was stirred under an atmosphere of argon at room temperature for 10 minutes. To the mixture was added a solution of thiophene-3-boronic acid (2.65 g, 20.7 mM) in ethylene glycol dimethly ether (20 ml), followed by aqueous 0.2 M sodium carbonate (10 ml). The mixture was refluxed under an atmosphere of argon for 12 hours. After cooling the mixture was poured into ice/water (50 ml), dichloromethane (50 ml) added and the organic layer separated. The organic layer was washed with ammonium acetate (2x25 ml), dried over sodium sulphate and the solvent removed *in vacuo*. Purification by column chromatography eluting with 10% ethyl acetate: hexane gave (R)-6,6'-di-(3-thienyl)-1,1'-bi-2-naphthol as a white solid (3.3 g, 81%).

Example 24

(R)-6,6'-di-(3-thienyl)-2,2'-ditrifluoromethanesulphonate-1,1'-binaphthyl

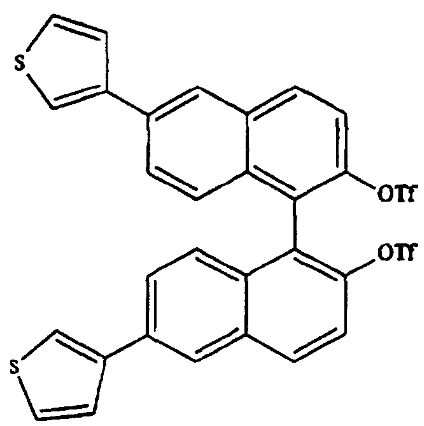

Trifluoromethanesulphonic anhydride (1.2 ml, 7.4 mM) was added to a stirred solution of (R)-6,6'-di-(3-thienyl)-1,1'-bi-2-naphthol (3.31g, 7.4 mM), 4-dimethylaminopyridine (13 mg, 0.1 mM), prepared according to Example 23, and 2,6-lutidine (2.2 ml, 18.5 mM) in dichloromethane (30 ml) at 0°C under an atmosphere of argon. The reaction mixture was allowed to warm to room temperature and stirred for a further 5 hours.

Saturated sodium bicarbonate solution (20 ml) was added to the reaction mixture to quench and the organic layer separated. The aqueous phase was washed successively with dichloromethane (2 x 30 ml). The combined organic extracts were dried over sodium sulphate and the solvent removed *in vacuo* to give an oil. Purification by column chromatography eluting with 5% ethyl acetate:hexane gave the title compound as a colourless oil (4.2g, 79%).

Example 25

(R)-6,6'-di-(3-thienyl)-2,2'-bis(diphenylphosphino)-1,1'-binaphthyl

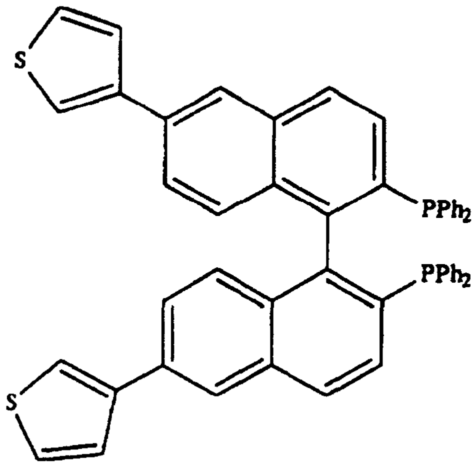

A solution of $NiCl_2$dppe (1.8g, 4.79 mM) in anhydrous dimethylformamide (15 ml) was degassed thoroughly using 7 pump/argon cycles. Diphenylphosphine (1.1 ml, 2.95 mM) was added and the red mixture aged at 100°C for 1 hour. In a separate flask were degassed (R)-6,6'-di-(3-thienyl)-2,2'-di-trifluoromethanesulphonate-1,1'-binaphthyl (2.1g, 2.94 mM), prepared according to Example 24, and 1,4-diazabicylco[2.2.2]octane (1.32g, 11.74 mM) in dimethylformamide (15 ml) and added to the nickel solution via canula. The resulting deep green solution was heated at 100°C, a further portion of diphenylphosphine (1.1 ml, 2.95 mM) added after 4 hours and continued heating for a further 16 hours. After cooling to room temperature the mixture was diluted with ethyl acetate (50 ml), poured onto 50 ml aqueous sodium cyanide (1.4g, 29.2 mM) and stirred vigorously for 1 hour. The layers were separated and the organic phase washed with water (3 x 25 ml), dried over anhydrous sodium sulphate and the solvents removed *in vacuo*. The resulting brown solid was then dissolved in anhydrous toluene (50 ml), treated with trichlorosilane (9.9 ml, 1.18 mM) and heated at reflux for 18 hours. The mixture was quenched by pouring carefully onto 2.0 M aqueous sodium hydroxide (50 ml) and stirring vigorously for 30 minutes. The layers were separated and the aqueous phase extracted with dichloromethane (3 x 50ml). The combined organic layers were dried over anhydrous sodium carbonate and the solvent removed *in vacuo.* Purification was effected by flash column chromatography (silica gel, ethyl acetate-hexane, 5%) to give the title compound as a white solid (1.1g, 48%).

Example 26

(R)-6,6'-di-phenyl-1,1'-bi-2-naphthol

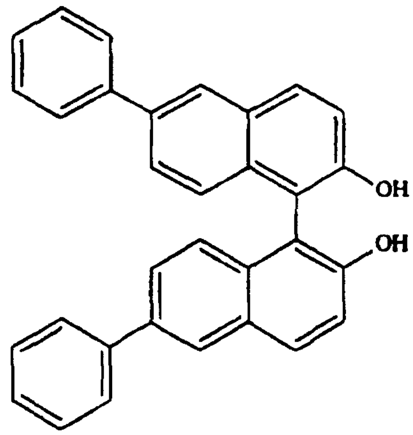

A solution of (R)-6,6'-dibromo-1,1'-bi-2-naphthol (3.07g, 6.92 mM), prepared according to the literature procedure in *J. Am. Chem. Soc*. 1979, **101,** 3035-3042, and tetrakis(triphenylphosphine)-palladium (0) (0.80g, 0.69 mM) in anhydrous toluene (30 ml) was stirred under an atmosphere of argon at room temperature. To the mixture was added dropwise phenyltrimethyl tin (5.0g, 20.7 mM). The mixture was refluxed under an atmosphere of argon for 16 hours. After cooling the mixture was filtered through a pad of celite and washed with ethyl acetate (2 x 30 ml). The solvent was removed *in vacuo* to yield a yellow oil. Purification by column chromatography eluting with dichloromethane gave the title compound as an off-white solid (1.92g, 63%).

Example 27

(R)-6,6'-di-phenyl-2,2'-di-trifluoromethanesulphonate-1,1'-binaphthyl

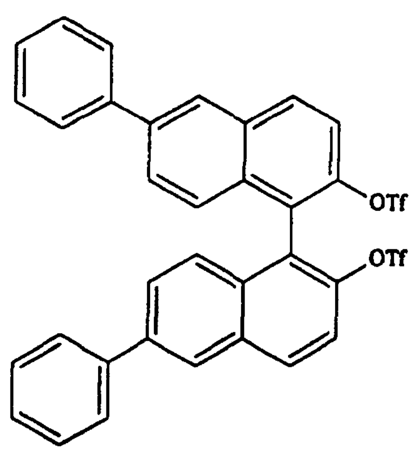

Trifluoromethanesulphonic anhydride (0.1 ml, 0.69 mM) was added to a stirred solution of (R)-6,6'-di-phenyl-1,1'-bi-2-naphthol (0.27g, 0.62 mM), prepared according to Example 26, 4-dimethylamino-pyridine (2.3 mg, 0.009 mM) and 2,6-lutidine (0.2 ml, 1.6 mM) in dichloromethane (5 ml) at 0°C under an atmosphere of argon. The reaction mixture

was allowed to warm to room temperature and stirred for a further 16 hours. Saturated sodium bicarbonate solution (10 ml) was added to the reaction mixture to quench and the organic layer separated. The aqueous phase was washed successively with dichloromethane (2 x 10 ml). The combined organic extracts were dried over sodium sulphate and the solvent removed *in vacuo* to give an oil. Purification by column chromatography eluting with 2% ethyl acetate: hexane gave the title compound as a colourless oil (0.36g, 85%).

Example 28

(R)-6,6'-di-phenyl-2,2'-bis(diphenyl-phosphino)-1,1'-binaphthyl

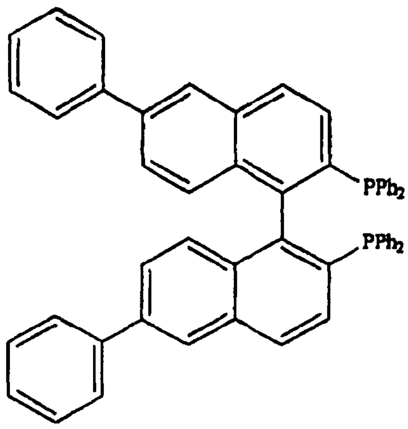

A solution of $NiCl_2$dppe (1.98g, 3.74 mM) in anhydrous dimethylformamide (10 ml) was degassed thoroughly using 7 pump/argon cycles. Diphenylphosphine (1.1 ml, 3.12 mM) was added and the red mixture aged at 100°C for 1 hour. In a separate flask were degassed (R)-6,6'-diphenyl-2,2'-di-trifluoromethanesulphonate-1,1'-binaphthyl (2.19g, 3.12 mM), prepared according to Example 27, and 1,4-diazabicyclo[2.2.2]octane (1.40g, 12.5 mM) in dimethylformamide (10 ml) and added to the nickel solution via canula. The resulting deep green solution was heated at 100°C, a further portion of diphenylphosphine (1.1 ml, 3.12 mM) added after 4 hours and continued heating for a further 16 hours. After cooling to room temperature the mixture was diluted with ethyl acetate (50 ml), poured onto 50 ml aqueous sodium cyanide (1.53g, 31.2 mM) and stirred vigorously for 1 hour. The layers were separated and the organic phase washed with water (3 x 30 ml), dried over anhydrous sodium carbonate and the solvents removed *in vacuo.* The resulting brown solid was then dissolved in anhydrous toluene (50 ml), treated with trichlorosilane (9.0ml, 1.1mM) and heated at reflux for 18 hr. The mixture was quenched by pouring carefully onto 2.0 M aqueous sodium hydroxide (50 ml) and stirring vigorously for 30 mins. The layers were separated and the aqueous phase extracted with Dichloromethane(3 x 50 ml). The combined organic layers were dried over anhydrous sodium carbonate and the solvent removed *in vacuo.* Purification was affected by flash column chromatography (silica gel, ethyl acetate-hexane, 5%) to give the title compound as a white solid (1.30g, 54%).

Example 29

(R)-6,6'-dihydroxymethyl-2,2'-dimethoxy-1,1-binaphthyl

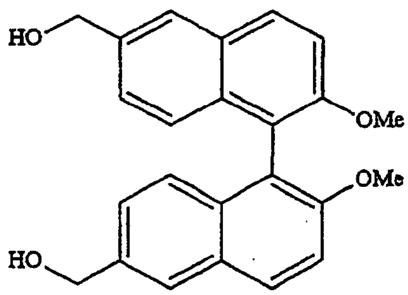

To a stirred solution of (R)-6,6'-dibromo-2,2'-dimethoxy-1,1'-binaphthyl (0.5g, 1.06mM) in tetrahydrofuran (5ml) at -78°C under an atmosphere of argon was added n-butyl lithium (1.7ml, 4.24mM). The reaction mixture was warmed to 0°C and the cooled back down to -78°C. To the reaction mixture was added a suspension of paraformaldehyde (0.20g) in tetrahydrofuran (2ml). The resulting reaction mixture was allowed to warm to room temperature and stirred for a further 0.5 hours after which water (5ml) was added dropwise to quench. Ethyl acetate (10ml) was added to the reaction mixture and the organic layer separated.
The aqueous phase was washed with ethyl acetate (2 x 10ml). The combined organic layers were dried over sodium sulphate and the solvent removed *in vacuo*. Purification by column chromatography eluting with 20% ethyl acetate:hexane gave the title compound as a white solid (0.27g, 68%).
Modifications of the 6,6'-methyl alcohol functionalities may now be carried out prior to transformation to the corresponding BINAPs, analogously to Examples 6 to 8, to give further compounds of the invention.

Example 30

(R) -6,6'-dicarbaldehyde-2,2'-dimethoxy-1,1-binaphthyl

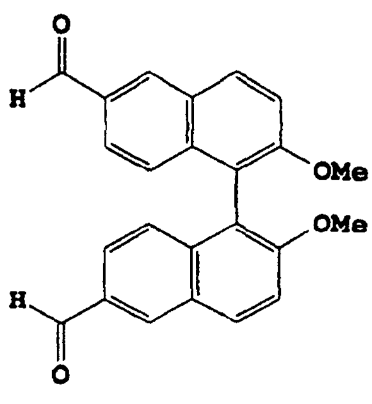

To a stirred solution of (R)-6,6'-dibromo-2,2'-dimethoxy-1,1'-binaphthyl (0.5g, 1.06mM) in tetrahydrofuran (5ml) at -78°C under an atmosphere of argon was added n-butyl lithium (1.7ml, 4.24mM). The reaction mixture was warmed to 0°C and then cooled back down to -78°C. The reaction mixture was added via cannula to a cooled (-78°C) stirred solution of dimethylformamide (1ml) under an atmosphere of argon. The resulting reaction mixture was allowed to warm to room temperature and stirred for a further 2 hours after which water (5ml) was added dropwise to quench. Ethyl acetate (10ml) was added to the reaction mixture and the organic layer separated. The aqueous phase was

washed with ethyl acetate (2 x 10ml). The combined organic layers were dried over sodium sulphate and the solvent removed *in vacuo*. Purification by column chromatography eluting with 10% ethyl acetate:hexane gave the title compound as a white solid (0.21g, 54%).

Modifications of the 6,6'-aldehyde functionalities may now be carried out prior to transformation to the corresponding BINAPs, analogously to Examples 6 to 8, to give further compounds of the invention.

Example 31

4-(6-(R)-2,2'-dimethoxy-1,1'-binaphthyl)-butanoic acid

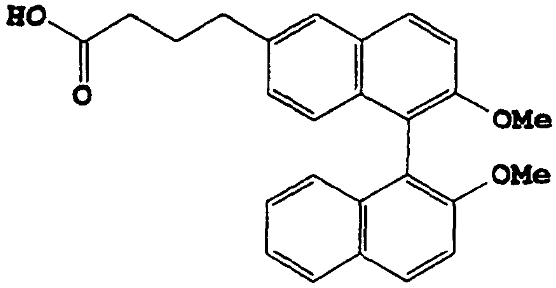

A solution of 4-(6-(R)-2,2'-dimethoxy-1,1'-binaphthyl)-ethylbutanoate (5) (210 mg, 0.49 mM) and 2N sodium hydroxide (13 ml) in THF (5 ml) was stirred at reflux for 15 hours. After cooling the reaction mixture was acidified to pH 4 by the addition of 2N HCl. The mixture was extracted with dichloromethane (3 x 20 ml). The combined organic layers were dried over sodium sulphate and the solvent removed *in vacuo.* Purification by column chromatography eluting with 40% ethyl acetate:hexane gave the title compound as a white solid in a yield of 180 mg (92%).

Example 32

(R)-6,5-(1,2-cyclohexan-3-one)-1,1'-bi-2-naphthol

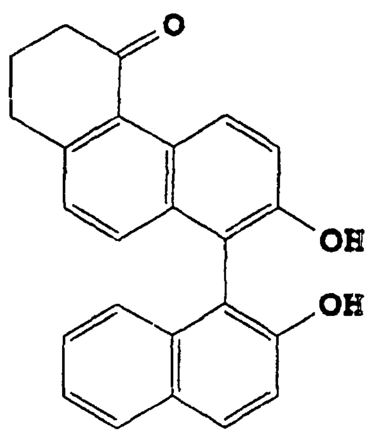

To a pre-cooled (-78°C) stirred, mixture of 4-(R)-(6)-2,2'-dimethoxy-1,1'-binaphthyl)-butanoic acid, prepared according to Example 31, (3.1 g, 7.24 mM) in dichloromethane (30 ml) under argon was added BBr3 (1.35 ml, 14.48 mM) dropwise. The reaction mixture was allowed to warm to room temperature and stirred for 2 hours. The reaction mixture was quenched by the dropwise addition of water (20 ml) and the resulting mixture extracted with dichloromethane (2 x 25 ml). The combined organic extracts were dried over sodium sulphate, filtered and the solvent removed *in*

*vacuo.* Purification by column chromatography eluting with 10% ethyl acetate:hexane gave the title compound as an off-white solid (1.6 g, 57%).

Example 33

(R)-6,5-(1,2-cyclohexane-3-one)-2,2'-bis(diphenylphosphino)-1,1'-binaphthyl

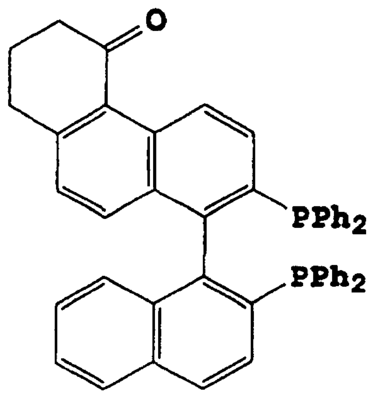

To a stirred solution of (R)-6,5-(1,2-cyclohexane-3-one)-1,1'-bi-2-naphthol (1.0 g, 2.8 mM), prepared according to Example 32, 2,6-lutidine (0.8 ml, 7.0 mM) and 4-dimethyl aminopyridine (6 mg, 0.04 mM) in dichloromethane (20 ml) at 0°C under an atmosphere of argon was added dropwise triflic anhydride (1.5 ml, 8.4 mM). The resulting reaction mixture was warmed to room temperature and stirred under argon for 5 hours. Water (20 ml) was added to the reaction mixture to quench it and the organic layer was separated. The aqueous layer was washed with dichloromethane (2 x 20 ml). The combined organic layers were dried over sodium sulphate and concentrated *in vacuo*. Purification by column chromatography eluting with 15% ethyl acetate:hexane gave the ditriflate of the starting material as an off-white solid (1.52 g, 87%).

To a stirred, degassed solution of $NiCl_2$dppe (870 mg, 1.62 mM) in anhydrous DMF (5 ml) under argon was added diphenylphosphine (1.0 ml, 2.73 mM). The resulting red mixture was stirred at 100°C for 1 hour. To this mixture at 100°C was added a degassed solution of the above triflate (0.9 g, 1.45 mM) and 1,4-diazabicyclo[2.2.2]octane (0.59 g, 5.54 mM) in dimethylformamide (DMF) (10 ml) via cannula. The resulting dark green solution was heated at 100°C for 4 hours after which a further portion of diphenylphosphine (1.0 ml, 2.73 mM) was added. Heating was continued for a further 16 hours. After cooling the mixture was diluted with ethyl acetate (25 ml), poured into 25 ml aqueous sodium cyanide (0.68 g, 13.6 mM) and stirred vigorously for 1 hour. The layers were separated and the organic layer washed with water (3 x 15 ml), dried over sodium sulphate and the solvents removed *in vacuo.* The resulting solid was dissolved in toluene (20 ml), treated with trichlorosilane (4.26 ml, 0.59 mM) and heated at reflux for 18 hours. The mixture was quenched by the careful addition of 2N aqueous sodium hydroxide (40 ml) and stirring vigorously for 30 mins. The layers were separated and the aqueous phase extracted with dichloromethane (3 x 20 ml). The combined organic layers were dried over sodium sulphate and the solvent removed *in vacuo.* Purification by column chromatography eluting with 10% ethyl acetate:hexane gave (R)-6,5-(1,2-cyclohexane-3-one)-2,2'-bis(diphenylphosphino)-1,1'-binaphthyl as a white solid (0.54 g, 51%).

Example 34

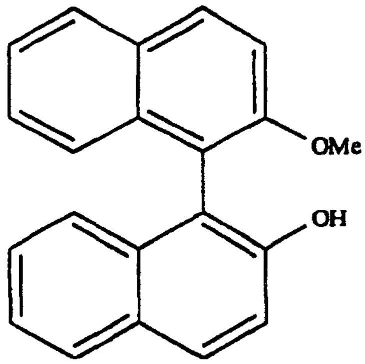

(R)-DimethoxyBINOL (3) (9046 g, 1 mole equivalent), prepared according to Example 1, in dichloromethane (10 vol) was cooled to -70°C. Boron tribromide (1.05 mole eq.) was added dropwise whilst maintaining the temperature at <-70°C. The reaction mixture was stirred at this temperature until no starting material remained (typically 2 hours, TLC:DCM). On reaction completion methanol (0.3 vol) was added dropwise, again maintaining the temperature at <-70°C. The reaction mixture was allowed to warm to 0°C, when water (6 vol) was added and the resulting mixture stirred for 30 minutes. The layers were separated and the aqueous layer was washed with DCM (2 x 1 vol). The organic extracts were combined, washed with sat. aq. sodium bicarbonate (3 vol) and dried over magnesium sulphate. Filtration, followed by concentration at reduced pressure and drying in a vacuum oven overnight at 40°C gave the product as a white solid in 99% overall yield (8541 g).

(In Examples 34-42, 1 volume corresponds to 1 litre of solvent per kilogram of substrate.)

Example 35

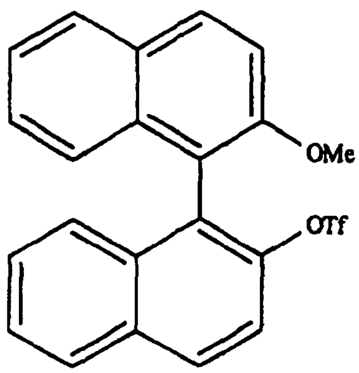

2,6-Lutidine (1.4 mole eq.) was added dropwise to a mixture of the mono-methoxy-BINOL derivative prepared according to Example 34 (8541 g, 1 mole eq.) and DMAP (5 mole %) in DCM (10 vol.), whilst maintaining the temperature in the range 0-5°C. Triflic anhydride (1.2 mole eq.) was then introduced dropwise, again keeping the temperature in the range of 0-5°C. The resulting mixture was allowed to warm to room temperature and stirred overnight, after which time the reaction was complete (TLC:DCM). Water (5 vol.) was added and the reaction mixture stirred for 10 minutes. The layers were separated and the aqueous layer washed with DCM (2 x 1 vol.). The combined organic extracts were washed with 2M hydrochloric acid (3 vol.), water (3 vol.). sat. aq. sodium bicarbonate (3 vol.) and dried over magnesium sulphate. Filtration, followed by concentration of the filtrate at reduced pressure gave a purple oil. Isopropyl alcohol (2 vol.) was introduced to the oil and the flask warmed at 40°C on a rotary evaporator until a yellow solution was obtained. The alcoholic solution was allowed to cool to room temperature and then cooled in ice. The resulting yellow solid was

removed by filtration and washed with ice-cooled isopropyl alcohol (1 x 0.5 vol.). After drying in a vacuum oven overnight at 40°C the desired product was obtained as a pale yellow solid in 88% overall yield (10715 g).

Example 36

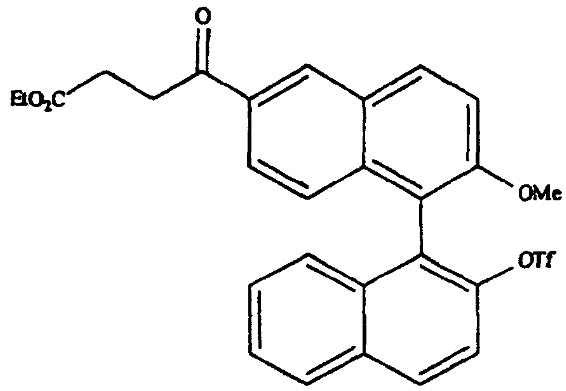

Aluminium chloride (2 mole eq.) was added portionwise to a solution of ethyl succinyl chloride (2 mole eq.) in DCE (6.5 vol.), whilst maintaining the temperature in the range 0-10°C. The resulting mixture was stirred until all the aluminium chloride had dissolved. To the resulting solution, a mixture of the mono-triflate BINOL derivative produced according to Example 35 (10751 g, 1 mole eq.) in DCE (2 vol.) was added dropwise whilst maintaining the temperature in the range 0-5°C. The cooling bath was removed and the reaction mixture stirred at room temperature overnight. The brown solution was warmed to 70°C and monitored every 10 minutes by TLC (30% ethyl acetate in hexanes) until the reaction was complete (typically 1 hour). The reaction mixture was allowed to cool to room temperature and then added dropwise, with care, onto ice water (8 vol.). The resulting mixture was stirred at room temperature for 30 minutes. The layers were separated and the aqueous layer washed with DCM (3 x 1 vol.). The combined organic extracts were washed with water (4 vol.) and sat. aq. sodium bicarbonate (4 vol.). Filtration, followed by concentration at reduced pressure to 5 volumes gave a dark brown solution. This solution was heated at 40°C for 1 hour with decolourising charcoal (0.25 wt) and hot filtered through glass fibre pads (this was necessary in order to obtain a solid product in the following reaction step). The charcoal was washed with DCM until all the product was reisolated. Concentration at reduced pressure gave a brown oil which was taken directly into the next step of the reaction (see Example 37).

Example 37

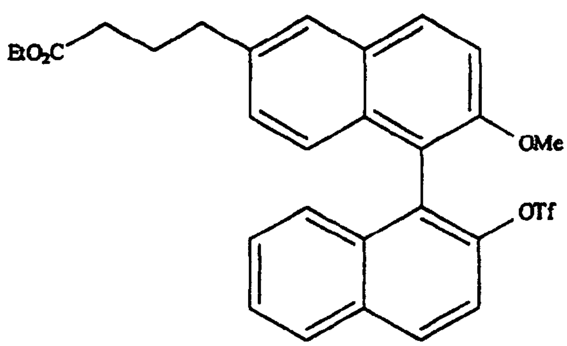

The crude product from Example 36 (1 mole eq.) was dissolved in trifluoroacetic acid (3 vol.) and cooled to <5°C. Triethylsilane (4 mole eq.) was added at such a rate so as to maintain the temperature at <30°C. The resulting

mixture was stirred at room temperature until no starting material remained (typically 2 hrs; TLC: 30% ethyl acetate in hexanes). Dichloromethane (3 vol.) was then added to the reaction mixture and the resulting solution added dropwise to ice/water (3 vol.). The layers were separated and the aqueous layer washed with DCM (2 x 1 vol.). The organic extracts were combined and washed with water (2 x 3 vol.), sat. aq. sodium bicarbonate (3 vol.), water (3 vol.) and dried over magnesium sulphate. Concentration at reduced pressure gave a brown oil to which was added isopropyl alcohol (1 vol.) and the resulting mixture stirred overnight at room temperature. The resulting yellow solid was isolated by filtration and recrystallised from boiling isopropyl alcohol (1 vol.). After drying the yellow solid thus obtained in a vacuum oven at 40°C overnight, the desired product was obtained in 63% overall yield (8617 g) over two stages.

Example 38

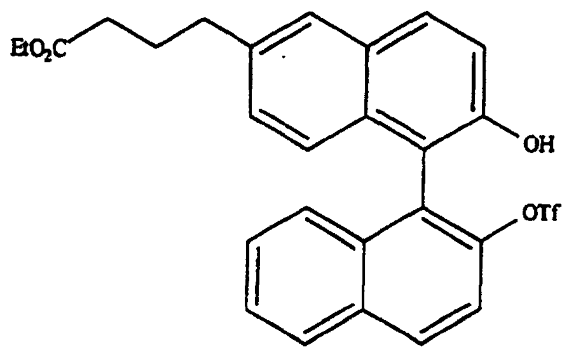

A solution of the product from Example 37 (8617 g, 1 mole eq.) in dichloromethane (10 vol.) was cooled to -20°C. Boron tribromide (2 mole eq.) was added dropwise maintaining the temperature at <-20°C. The reaction mixture was stirred at this temperature until no starting material remained (typically 2 hours, TLC:DCM). On reaction completion methanol (3 vol.) was added dropwise, again maintaining the temperature at <-20°C. The reaction mixture was allowed to warm to >0°C when water (6 vol.) was added and the resulting mixture stirred for 30 minutes. The layers were separated and the aqueous layer washed with DCM (2 x 1 vol.). The organic extracts were combined, washed with sat. aq. sodium bicarbonate (3 vol.) and dried over magnesium sulphate. Filtration, followed by concentration at reduced pressure gave the crude product as a pale brown oil. A 4L sinter funnel was slurry packed with silica, 1.9 kg, in DCM and sucked dry. The crude reaction mixture (500g) was dissolved in DCM (700ml) and loaded onto the top of the column. The product was separated from baseline material by eluting with DCM (10 x 2L). Concentration at reduced pressure gave the pure product as a brown oil in 90% overall yield (7539 g).

Example 39

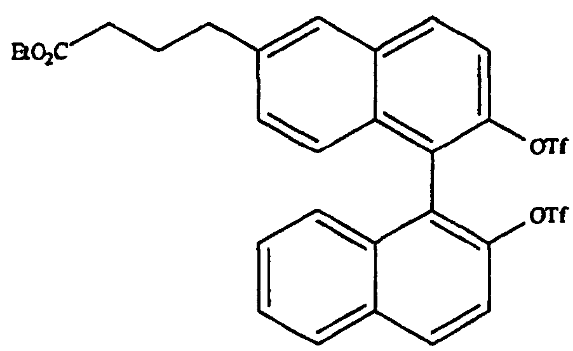

2,6-Lutidine (1.4 mole eq.) was added dropwise to a mixture of the mono-methoxy-BINOL product from Example 38 (7539 g, 1 mole eq.) and DMAP (5 mole %) in DCM (10 vol.), whilst maintaining the temperature in the range 0-5°C. Triflic anhydride (1.2 mole eq.) was then introduced dropwise again keeping the temperature in the range of 0-5°C. The resulting mixture was allowed to warm to room temperature and stirred overnight, after which time the reaction was complete (TLC:DCM). Water (5 vol.) was added and the reaction mixture stirred for 10 minutes. The layers were separated and the aqueous layer washed with DCM (2 x 1 vol.). The combined organic extracts were washed with 2M hydrochloric acid (3 vol.), water (3 vol.), sat. aq. sodium bicarbonate (3 vol.) and dried over magnesium sulphate. Filtration, followed by concentration of the filtrate at reduced pressure gave a brown oil in 98% overall yield (9319 g), which was taken directly to the next stage without further purification.

Example 40

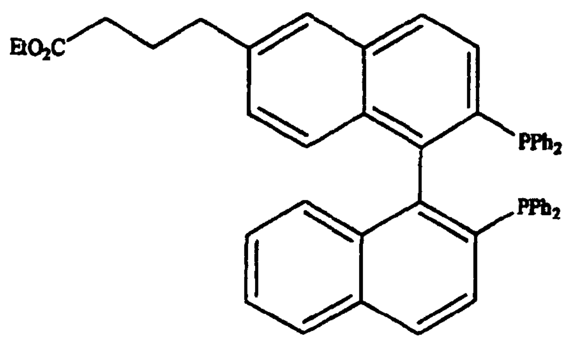

To a stirred suspension of $NiCl_2$dppe (0.2 mole eq.) in DMF (3 vol.) was added a 50% solution of diphenylphosphine in DMF (0.56 mole eq. of diphenylphosphine) and the mixture heated to 100°C. After 45 minutes at this temperature a solution of the ditriflate product form Example 39 (5843 g, 1 mole eq.) and DABCO (4 mole eq.) in DMF (5 vol.) was added. After a further 1.3 and 6 hours at 100°C, additional portions of the diphenylphosphine solution (0.56 mole eq. of diphenylphosphine) were added and the resulting mixture stirred at 100°C overnight. Once the reaction was complete (TLC 20% ethyl acetate in hexanes) the reaction mixture was allowed to cool to room temperature and poured carefully onto vigorously stirred ice/water (10 vol.). Stirring was maintained for 30 minutes and the precipitated solids collected by filtration), washed with water (4 x 2 vol.) and sucked dry. The residual solids were then dissolved in DCM (5 vol.) and the solution left to stand overnight. Any water was separated and the organic solution filtered through Celite. After drying the solution over magnesium sulphate, concentration at reduced pressure gave a brown oil. Purification of the crude oil was achieved by flash column chromatography. A 4L sinter funnel was packed with silica (1.8 kg) in DCM and sucked dry. The crude reaction mixture (250 g) was dissolved in DCM (500 ml) and loaded onto the column. Eluting with: 5% ethyl acetate in hexanes (5 x 2L) isolated an unidentified impurity ($R_f$ 0.75), eluting with 20% ethyl acetate in hexanes (7 x 2L) gave the desired product ($R_f$ 0.57) and eluting with 50% ethyl acetate in hexanes (5 x 2L) gave the phosphine oxides ($R_f$ 0.1). Concentration at reduced pressure gave the desired product in 66% overall yield (4291 g), as a yellow solid, and the phosphine oxides (mono and di-mixture), 1.3 Kg overall as a brown oil.

Example 41

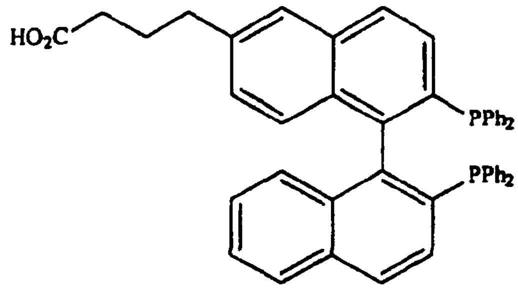

To a solution of the BINAP ester product from Example 40 (4571 g, 1 mole eq.) in THF (6 vol.) was added lithium hydroxide (2 mole eq.) in water (3 vol.) and the mixture heated at reflux overnight. Once the reaction was complete, (TLC: 50% ethyl acetate in hexanes), it was allowed to cool to room temperature and cone. hydrochloric acid added until the pH = 1. Dichloromethane (5 vol.) was added and the mixture stirred for 10 minutes. The layers were separated and the aqueous layer washed with DCM (2 x 1 vol.). The combined organic extracts were washed with water (2 x 2 vol.) and dried over magnesium sulphate. Filtration, and concentration at reduced pressure gave the crude product as a pale yellow oil. Addition of methanol (4 vol.) and stirring at room temperature for 2 hours yielded a white precipitate. The precipitate was isolated by filtration and recrystallised from methanol (30-35 vol.). The mother liquors were concentrated at reduced pressure and recrystallised once more to give a second crop. After drying the solids in a vacuum oven overnight at 40°C the desired product was obtained as a white solid in 63% overall yield (2782 g).

Example 42

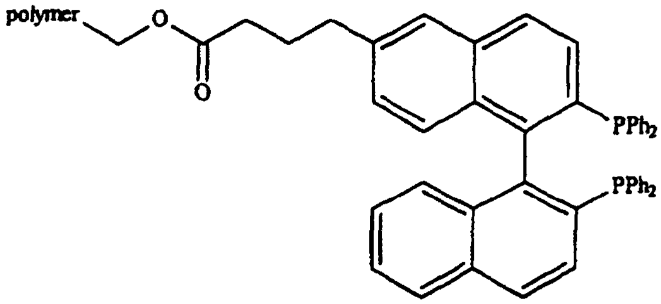

A mixture of chloromethyl-polystyrene resin (1 mole eq.), the BINAP acid product from Example 41 (2129 g, 1.1 mole eq.), caesium carbonate (2.2 mole eq.) and potassium iodide (0.5 eq.) in DMF (17 vol.) was heated at 80°C for 60 hours. The reaction mixture was allowed to cool to 50°C and filtered through a sinter funnel. The residual solids were washed in the sinter funnel with DMF (1 vol.) and water at 40°C (2 x 2 vol.). The remaining solids were then repeatedly slurried in water at 40°C (2 vol.) until the pH of the filtrate was neutral. The solids were then slurried with THF (8 vol.), MeOH (2 x vol.) and dried in the vacuum oven at 40°C overnight to give the desired polymer bound BINAP as an off-white powder (5190 g). Loadings ranged from 0.32-0.45 mmol/g.

Example 43

Analysis of hydrogenation products

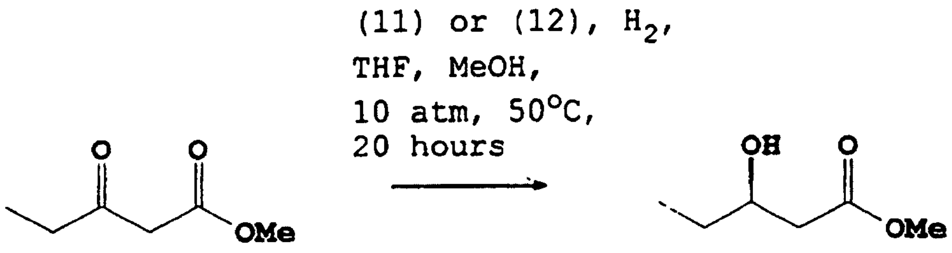


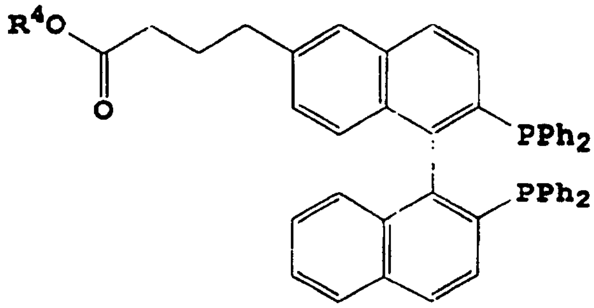

(11), (12)

| Catalyst | $R^4$ | Optical purity of product | Yield |
|---|---|---|---|
| (11) (R-enantiomer) | $CH_3CH_2$ | 98.9% | 100% |
| (12) (R-enantiomer) | H | 98.8% | 100% |
| (R) -BINAP-$RuBr_2$[1] | | 99% | 100% |

[1] Comparative Example - literature values taken from Genet, J.P., Pinel, C., Ratovelomanana-Vidal, V., Mallart, S., Pfister, X., Bischoff, L., Cano de Andrade, M.C., Darses, S., Galopin, C., Laffitte, J.A., *Tet. Asymm.*, 1994, **5(4),** 675.

The non $C_2$-symmetric catalysts of the invention show very similar yields and essentially identical enantiomeric excesses to the prior art catalyst.

Example 44

Some typical values demonstrating the comparison of support-bound catalysts according to the invention with prior art catalysts

| Solution phase BINAP catalysts (typical values)[2] | Support-bound BINAP catalysts of the invention |
|---|---|
| 1-2 mol% catalyst used | 2 mol% catalyst used |
| 4-20 atm. hydrogen used | 10 atm. hydrogen used |

[2] Values for the solution phase BINAP were taken from Genet, J.P., Pinel, C., Ratovelomanana-Vidal, V., Mallart, S., Pfister, X., Bischoff, L., Cano De Andrade, M.C., Darses, S., Galopin, C., Laffitte, J.A., *Tet. Asymm.*, 1994, **5(4),** 675.

(continued)

| Solution phase BINAP catalysts (typical values)[2] | Support-bound BINAP catalysts of the invention |
|---|---|
| 40-80°C temperature used | 35-50°C temperature used |
| 30 min-16 hours reaction time | 16-24 hours reaction time (36 hours when reusing catalyst for second time) |
| 70-100% purities | 80-100% purities, 100% conversions |
| 75->99% optical purity | 64-<96.9% optical purity |

[2] Values for the solution phase BINAP were taken from Genet, J.P., Pinel, C., Ratovelomanana-Vidal, V., Mallart, S., Pfister, X., Bischoff, L., Cano De Andrade, M.C., Darses, S., Galopin, C., Laffitte, J.A., *Tet. Asymm.*, 1994, **5(4),** 675.

It can be seen from the above values that the support-bound catalysts of the invention can be used under very similar conditions to the prior art catalysts, and with similar results.

Example 45

Comparison of support bound catalyst of the invention with prior art catalyst

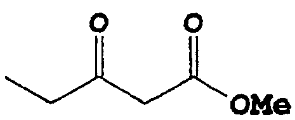
(13)

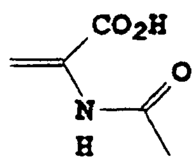
(14)

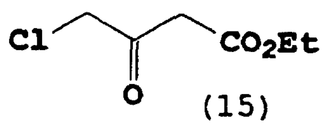
(15)

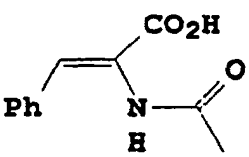
(16)

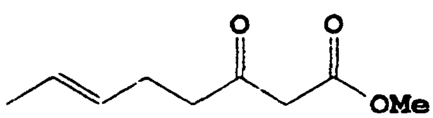
(17)

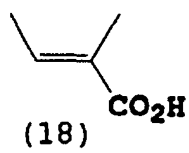
(18)

| Substrate | Conditions and results using support bound catalyst (10) of the invention (2 mol%) | Comparative conditions and results using prior art solution phase (R) or (S) BINAP-RuBr$_2$ catalyst (2 mol%)[3] |
|---|---|---|
| 13 | THF:MeOH (1:1), 10 atm., 50°C, 18 hours, 100% conv., 96.9% ee | MeOH, 20 atm., 40°C, 16 hours, 100% yield, >99%ee |
| 14 | THF, trace MeOH, 10 atm., 35°C, 23 hours, 100% conv. 64.5% ee | THF:EtOH (1:1), 20 atm., 48-72 hours, 1 mol% catalyst, 70% yield, 75% ee |
| 15 | THF:EtOH (1:1), 10 atm., 50°C, 24 hours, 100% conv., 94.9% ee | EtOH, 70 atm., 1 hours, 93°C, 100% yield, 89% ee |
| 16 | THF, trace MeOH, 10 atm., 35°C, 23 hours, 100% conv. 68% ee | N/A |

[3] Values for the solution phase BINAP were taken from Genet, J.P., Pinel, C., Ratovelomanana-Vidal, V., Mallart, S., Pfister, X., Bischoff, L., Cano De Andrade, M.C., Darses, S., Galopin, C., Laffitte, J.A., *Tet. Asymm.,* 1994, **5(4),** 675.

(continued)

| Substrate | Conditions and results using support bound catalyst (10) of the invention (2 mol%) | Comparative conditions and results using prior art solution phase (R) or (S) BINAP-RuBr$_2$ catalyst (2 mol%)[3] |
|---|---|---|
| 17 | THF:MeOH (1:1), 10 atm., 50°C, 20 hours, 100% conv.* 89% ee | MeOH, 4 atm., 80°C, 25 min, 95% yield,***99% ee |
| 18 | THF:MeOH (1:1), 10 atm., 50°C, 20 hours, 100% conv** | MeOH, 4 atm., 20°C, 24 hours, 1 mol% catalyst, **** 100% yield, 90% ee |

* complete hydrogenation of the olefin and ketone was observed.

** 50% of the corresponding methyl ester was observed.

*** less than 5% of the corresponding β-hydroxyester was observed.

**** (R)-BINAP-Ru(all)$_2$ catalyst used.

[3] Values for the solution phase BINAP were taken from Genet, J.P., Pinel, C., Ratovelomanana-Vidal, V., Mallart, S., Pfister, X., Bischoff, L., Cano De Andrade, M.C., Darses, S., Galopin, C., Laffitte, J.A., *Tet. Asymm.,* 1994, **5(4),** 675.

The above results demonstrate that the support-bound catalysts of the invention produce very similar results in terms of yield and enantiomeric excess as do the prior art catalysts. However, work up and purification of the products is significantly easier when the catalysts of the invention are used.

## Claims

**1.** Compounds of general formula (I)

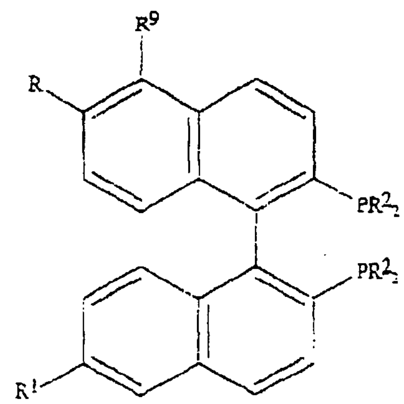

(I)

wherein

R denotes $C_{1-5}$ alkyl, $C_{1-6}$ alkenyl, $C_{1-6}$-alkynyl or phenyl, wherein the $C_{1-6}$ alkyl and phenyl groups may optionally be substituted by one or more substituents which may include F, Cl, Br, $NO_2$, amino, naphthalene, anthracene, biphenyl, $C_{1-6}$ alkyl, $CF_3$, CN, OH, O-$C_{1-6}$ alkyl, $CO_3H$, CHO, NHCO($C_{1-6}$ alkyl), $CO_2$($C_{1-6}$ alkyl), N($C_{1-6}$ alkyl)CO, benzyl, $C_{5-6}$ cyclic ethers or $C_{2-4}$ unsaturated hydrocarbon groups, and wherein the $C_{1-6}$ alkyl group may optionally include one or more intervening hetercatoms or aryl groups in the chain or R denotes CN, $CO_2NHR^3$, $(CH_2)_nOR^3$, $CO_2R^3$, benzyl, heterocyclic groups such as thiophene, furan, pyridine, pyrimidine, quinoline, benzofuran, benzothiophene, pyrrole, imidazole, isoquinoline or indole, wherein the heterocyclic groups may be optionally substituted by one or more ether or $C_{1-6}$ alkyl groups, or Y-X-$R^4$;

$R^1$ denotes R or H;

R2 denotes phenyl, phenyl substituted by one or more $C_{1-7}$ alkyl groups, O-$C_{1-6}$ alkyl groups and/or halogen atoms, or $R^2$ denotes a $C_{3-7}$ cyclic aliphatic hydrocarbon group;

$R^9$ denotes H or together with R forms a 5, 6 or 7 membered hydrocarbon ring, optionally substituted by one or more C=O, OH or amine groups;

Y denotes a straight or branched aliphatic chain, optionally incorporating one or more aromatic hydrocarbon group(s) or ether linkages in the chain, or an aromatic hydrocarbon group;

X denotes $CH_2$, $CO_2$, O, CONH, NH, $CONR^2$, $NR^2$ or a valence bond;

R3 denotes H, $C_1$-$C_{10}$ alkyl, benzyl or phenyl; and

$R^4$ denotes H, $C_1$-$C_6$ alkyl, an insoluble support, or a spacer group attached to an insoluble support;

and all enantiomers, mixtures, including racemic mixtures, and diastereomers thereof.

**2.** Compounds as claimed in claim 1 wherein

$R^2$ denotes phenyl;

X denotes CONH or $CO_2$;

Y denotes $(CH_2)_n$ wherein n denotes 2 to 4; and

$R^1$ is identical to R.

**3.** Compounds as claimed in claim 1 or claim 2 wherein

$R_2$ denotes phenyl;

$R_4$ denotes an insoluble support or a spacer group attached to an insoluble support;

X denotes CONH or $CO_2$; and

Y denotes $(CH_2)_n$ wherein n denotes 2 to 4.

**4.** Compounds as claimed in any of claims 1 to 3 of formula

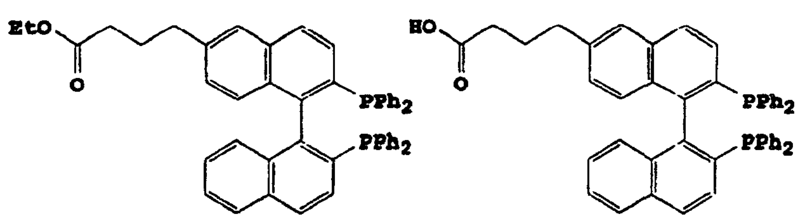

.

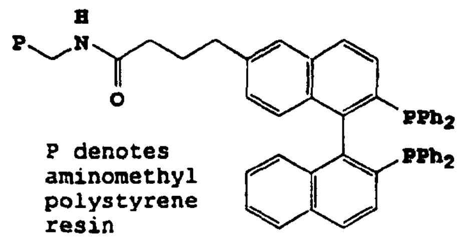

P denotes aminomethyl polystyrene resin

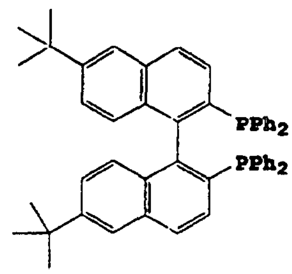

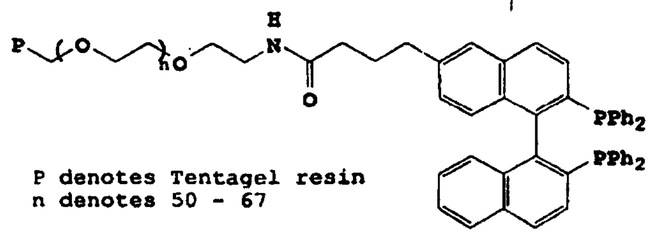

P denotes Tentagel resin
n denotes 50 - 67

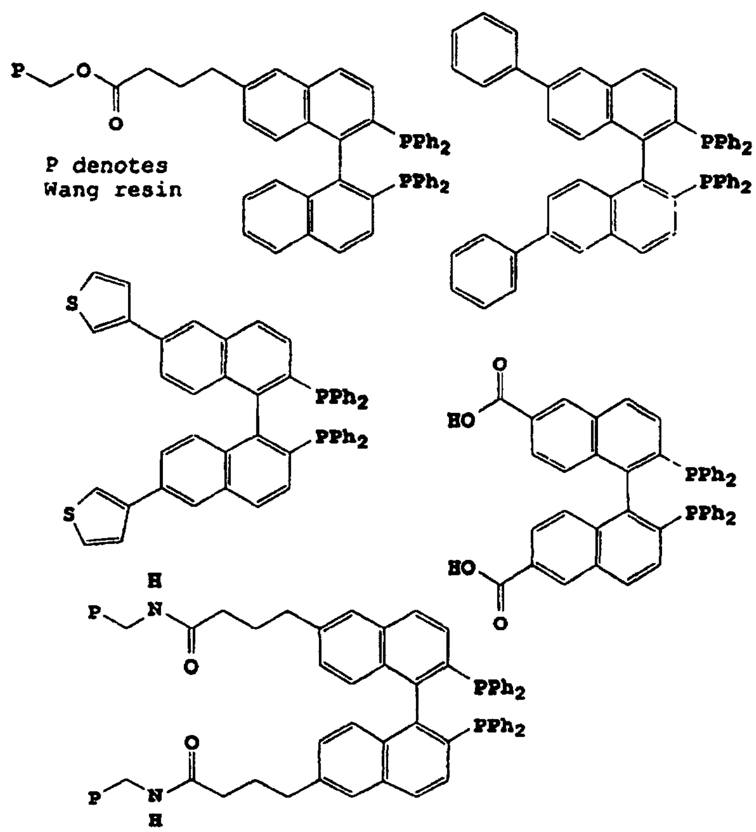

P denotes Wang resin

5. Compounds as claimed in any of claims 1 to 3 wherein the support is polystyrene-divinyl benzene co-polymer (Merrifield Resin), polystyrene resin, polyamide, aminomethylated polystyrene resin, Wang resin, aminomethylated Tentagel resin, polyamide-kieselguhr composites, polyhipe, cotton or paper.

6. Compounds as claimed in any of claims 1 to 5 which consist of a single enantiomer.

7. Complexes comprising a compound of formula (I) as claimed in any of claims 1 to 6 complexed to a transition metal.

8. Complexes as claimed in claim 7 wherein the transition metal is rhodium, ruthenium, palladium, iridium, nickel,

cobalt or molybdenum.

**9.** Complexes as claimed in claim 7 or claim 8 wherein the transition metal is ruthenium, rhodium or palladium.

**10.** Complexes as claimed in any of claims 7 to 9 of empirical formula $LRuBr_2$, wherein L denotes a compound of general formula (I) as claimed in any of claims 1 to 6.

**11.** Complexes as claimed in claim 10 wherein L is a compound of formula (II)

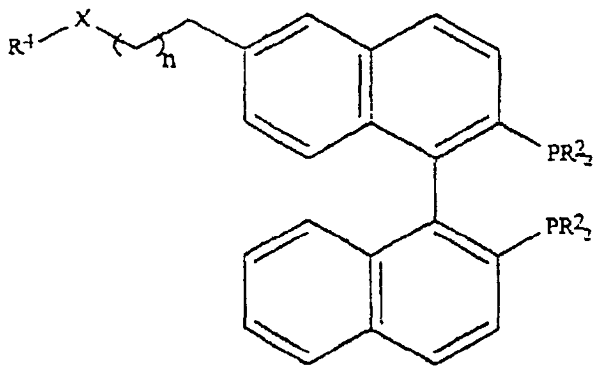

(II)

wherein

X denotes $CO_2$, O, NH, CONH, $CH_2$ or a valence bond;

n denotes 0-9; and

$R^2$ and $R^4$ are as defined in any of claims 1 to 3.

**12.** A process for the preparation of compounds as claimed in any of claims 1 to 6 wherein $R^1$ denotes Y-X-$R^4$ and $R^4$ denotes an insoluble support, which comprises linking a compound of formula (I) wherein $R^4$ does not denote an insoluble support to an insoluble support.

**13.** A process for the preparation of compounds as claimed in any of claims 1 to 6 which comprises reacting a compound of formula (V)

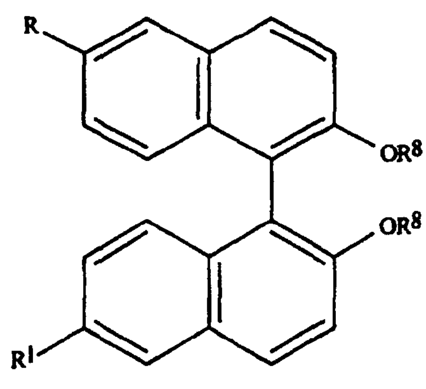

(V)

wherein

$OR^8$ denotes a leaving group which may be displaced by $HPR^2_2$; and
R and $R^1$ are as defined in any of claims 1 to 5;

with $HPR^2{}_2$,
wherein $R^2$ is as defined in any of claims 1 to 3.

**14.** Compounds of formula (V)

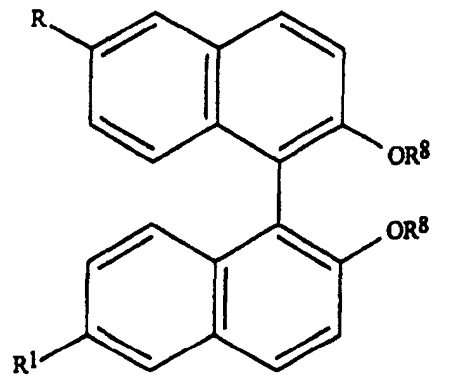

(V)

wherein

$OR^8$ denotes a leaving group which may be displaced by $HPR^2_2$; and
R and $R^1$ are as defined in any of claims 1 to 5.

**15.** Compounds as claimed in claim 14 wherein $R^8$ denotes $SO_2CF_3$.

**16.** Compounds of formula (III)

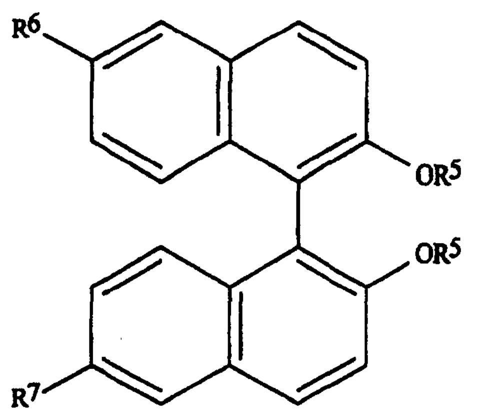

(III)

wherein

$R^5$ denotes any alkyl group which directs substitution to the 6 position;

$R^6$ denotes Cl, a straight or branched acyl or non-acyl aliphatic chain, optionally terminating in an acid functionality and optionally incorporating one or more aromatic hydrocarbon, ether, ester or amide groups within the chain or terminating the chain; or $R^6$ denotes a phenyl group, optionally substituted by one or more F, Cl, Br, $NO_2$, amino, naphthalene, anthracene, biphenyl, $C_{1-6}$ alkyl, $CF_3$, CN, OH, O-$C_{1-6}$ alkyl, $CO_2H$, CHO, NHCO($C_{1-6}$ alkyl), $CO_2$($C_{1-6}$ alkyl), N($C_{1-6}$ alkyl)CO, benzyl, $C_{5-6}$ cyclic ethers or $C_{2-4}$ unsaturated hydrocarbon groups, or $R^6$ denotes a heterocyclic group such as thiophene, furan, pyridine, pyrimidine, quinoline, benzofuran, benzothiophene, pyrrole, imidazole, isoquinoline or indole, wherein the heterocyclic groups may be optionally substituted by one or more ether or $C_{1-6}$ alkyl groups; and

$R^7$ denotes $R^6$ or H;

with the provisos that

a) when $R^5$ denotes methyl and $R^7$ denotes H, then $R^6$ does not denote $-CO(CH_2)_3COOCH_3$, $-CO(CH_2)_3COOH$ or $-(CH_2)_4COOH$; and
b) when $R^7$ denotes $R^6$, then $R^6$ does not denote $C_{1-30}$ alkyl, $C_{6-18}$ aryl or $C_{7-30}$ aralkyl.

**17.** Compounds as claimed in claim 16 wherein $R^5$ denotes $C_{1-7}$ alkyl or a $C_{3-7}$ cyclic aliphatic group.

**18.** Compounds as claimed in claim 16 or claim 17
wherein $R^5$ denotes$(CH_2)_{0-6}CH_3$

**19.** A process for the preparation of compounds as claimed in any of claims 1 to 6 which comprises converting the $OR^5$ groups in a compound of formula (III) as claimed in any of claims 16 to 18 into leaving groups which may be displaced by $HPR^2_2$, reacting the resultant product with $HPR^2_2$, wherein $R^2$ is as defined in any of claims 1 to 3, and, if necessary, performing synthetic chemistry to convert the $R^6$ and $R^7$ groups into R and $R^1$ groups respectively.

**20.** A process for the preparation of compounds as claimed in any of claims 16 to 18 which comprises reacting a compound of formula (IV)

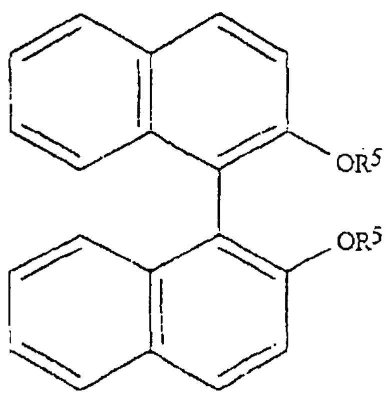

(IV)

wherein $R^5$ is as defined in any of claims 16 to 18, with a compound of formula $R^6$Hal,
wherein Hal denotes Cl, Br or I and $R^6$ is as defined in any of claims 16 to 16, with the proviso that $R^6$ does not denote C1.

**21.** Use of complexes as claimed in any of claims 7 to 11 as catalysts in asymmetric reduction reactions.

**Patentansprüche**

**1.** Verbindungen der allgemeinen Formel (1)

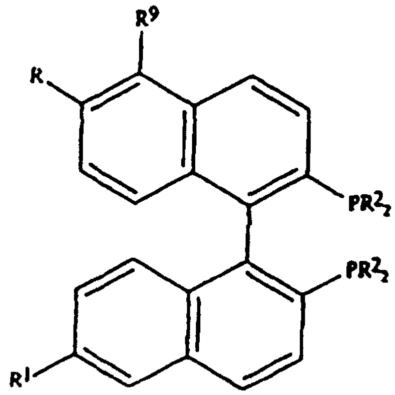

(I)

wobei

R einen $C_{1-6}$-Alkyl-, $C_{1-6}$-Alkenyl-, $C_{1-6}$-Alkinyl- oder Phenylrest bedeutet, wobei die $C_{1-6}$-Alkyl- und Phenylgruppen wahlweise durch einen oder mehrere Substituenten substituiert sein können, die F, Cl, Br, $NO_2$, Aminogruppen, Naphtalengruppen, Anthracengruppen, Biphenylgruppen, $C_{1-6}$-Alkylgruppen, $CF_3$, CN, OH, O-$C_{1-6}$-Alkylgruppen, $CO_2H$, CHO, NHCO ($C_{2-6}$-Alkyl)-Gruppen, $CO_2$($C_{1-6}$-Alkyl)-Gruppen, N($C_{1-6}$-Alkyl) CO-Gruppen, Benzylgruppen, zyklische $C_{5-6}$-Ether oder ungesättigte $C_{2-4}$-Kohlenwasserstoffgruppen enthalten können, und wobei die $C_{1-6}$-Alkylgruppe wahlweise ein oder mehrere dazwischenliegende Heteroatome oder Arylgruppen in der Kette enthalten kann, oder wobei R CN, $CO_2NHR^3$, $(CH_3)_nOR^3$, $CO_2R^3$, Benzylgruppen, heterozyklische Gruppen, wie Thiophen, Furan, Pyridin, Pyrimidin, Chinolin, Benzofuran, Benzothiophen, Pyrrol, Imidazol, Isochinolin oder Indol bedeutet, wobei die heterozyklischen Gruppen wahlweise durch eine oder mehrere Ether- oder $C_{1-6}$-Alkylgruppen oder Y-X-$R^4$ substituiert sein können,

$R^1$ R oder H bedeutet,

$R^2$ einen Phenylrest, einen Phenylrest bedeutet, der durch ein oder mehrere $C_{1-7}$-Alkylgruppen, O-$C_{1-6}$-Alkylgruppen und/oder Halogenatome substituiert ist, oder $R^2$ eine zyklische aliphatische $C_{3-7}$-Kohlenwasserstoffgruppe bedeutet,

$R^9$ H bedeutet oder zusammen mit R einen 5-, 6- oder 7-gliedrigen Kohlenwasserstoffring bildet, der wahlweise durch eine oder mehrere C=O, OH- oder Amingruppen substituiert ist,

Y eine unverzweigte oder verzweigte aliphatische Kette, die wahlweise eine oder mehrere aromatische Kohlenwasserstoffgruppen oder Etherbindungen in der Kette beinhaltet oder eine aromatische Kohlenwasserstoffgruppe bedeutet,

X $CH_2$, $CO_2$, O, CONH, NH, $CONR^2$, $NR^2$ oder eine Valenzbindung bedeutet,

$R^3$ H, einen $C_1$-$C_{10}$-Alkylrest, einen Benzylrest oder einen Phenylrest bedeutet, und

$R^4$ H, einen $C_1$-$C_6$-Alkylrest, einen unlöslichen Träger oder eine Abstandsgruppe bedeutet, die mit einem unlöslichen Träger verbunden ist,

und alle Enantiomere, Mischungen, einschließlich racemischer Mischungen, und Diastereomere davon.

**2.** Verbindungen nach Anspruch 1, wobei

$R^2$ einen Phenylrest bedeutet,

X CONH oder $CO_2$ bedeutet,

Y $(CH_2)_n$ bedeutet, wobei n 2 bis 4 bedeutet, und

$R^1$ identisch mit R ist.

**3.** Verbindungen nach Anspruch 1 oder 2, wobei

$R^2$ einen Phenylrest bedeutet,

$R^4$ einen unlöslichen Träger oder eine Abstandsgruppe bedeutet, die mit dem unlöslichen Träger verbunden ist,

X CONH oder $CO_2$ bedeutet, und

Y $(CH_2)_n$ bedeutet, wobei n 2 bis 4 bedeutet.

**4.** Verbindungen nach einem der Ansprüche 1 bis 3 mit der Formel

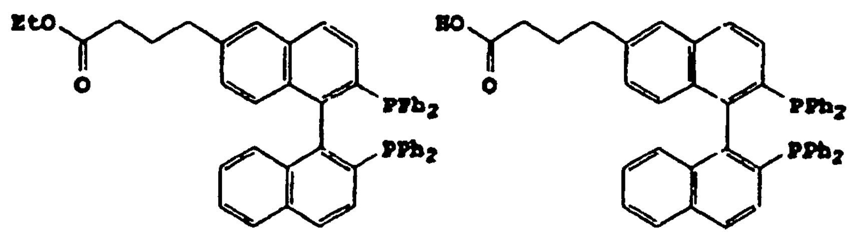

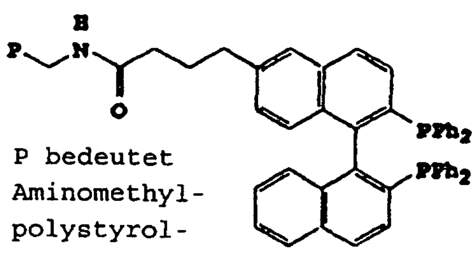

P bedeutet Aminomethyl-polystyrol-harz

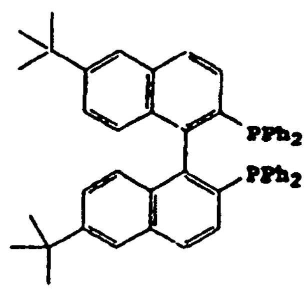

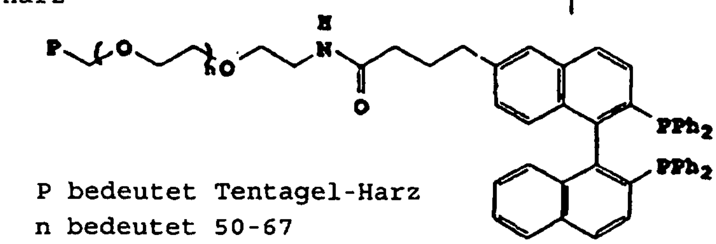

P bedeutet Tentagel-Harz
n bedeutet 50-67

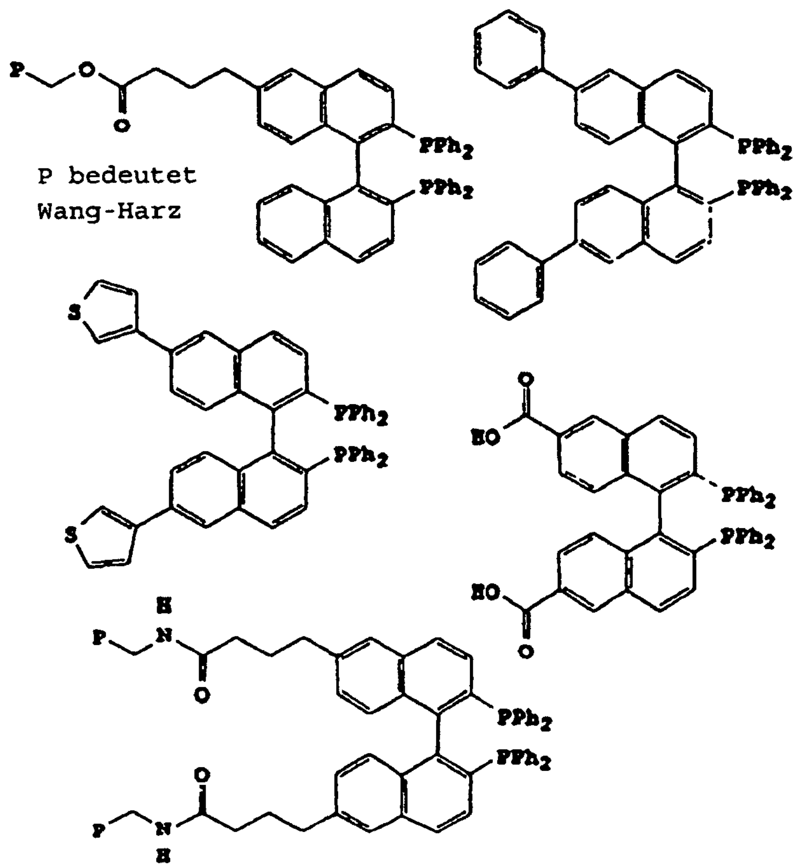

P bedeutet Wang-Harz

**5.** Verbindungen nach einem der Ansprüche 1 bis 3, wobei der Träger ein Polystyrol-Divinylbenzol-Copolymer (Merrifield Harz), ein Polystyrolharz, ein Polyamid, ein aminomethyliertes Polystyrolharz, ein Wang-Harz, ein aminomethyliertes Tentagel-Harz, Polyamid-Kieselgurverbundstoffe, Polyhipe, Baumwolle oder Papier ist.

**6.** Verbindungen nach einem der Ansprüche 1 bis 5, die aus einem einzigen Enantiomer bestehen.

**7.** Komplexe, die eine Verbindung mit Formel (I) nach einem der Ansprüche 1 bis 6, die mit einem Übergangsmetall komplexiert sind.

**8.** Komplexe nach Anspruch 7, wobei das Übergangsmetall Rhodium, Ruthenium, Palladium, Iridium, Nickel, Kobalt oder Molybdän ist.

**9.** Komplexe nach Anspruch 7 oder Anspruch 8, wobei das Übergangsmetall Ruthenium, Rhodium oder Palladium ist.

**10.** Komplexe nach einem der Ansprüche 7 bis 9 mit der empirischen Formel $LRuBr_2$, wobei L eine Verbindung mit der allgemeinen Formel (I) nach einem der Ansprüche 1 bis 6 bedeutet.

**11.** Komplexe nach Anspruch 10, wobei L eine Verbindung mit der Formel (II) ist

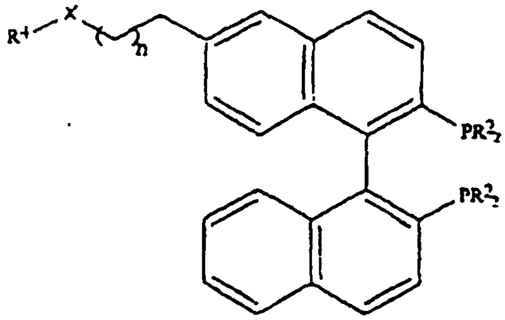

(II)

wobei

X $CO_2$, O, NH, CONH, $CH_2$ oder eine Valenzbindung bedeutet,

n 0 bis 9 bedeutet, und

$R^2$ und $R^4$ wie in einem der Ansprüche 1 bis 3 definiert sind.

**12.** Verfahren für die Herstellung von Verbindungen nach einem der Ansprüche 1 bis 6, wobei $R^1$ Y-X-$R^4$ bedeutet und $R^4$ einen unlöslichen Träger bedeutet, das das Verbinden einer Verbindung mit Formel (I), bei der $R^4$ keinen unlöslichen Träger bedeutet, mit einem unlöslichen Träger umfaßt.

**13.** Verfahren für die Herstellung von Verbindungen nach einem der Ansprüche 1 bis 6, das die Reaktion einer Verbindung mit Formel (V)

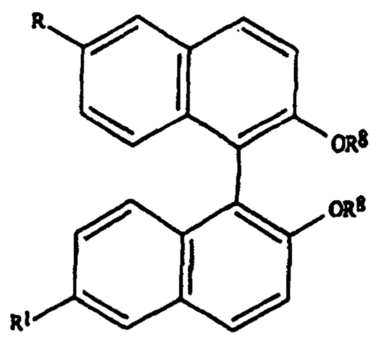

(V)

wobei

$OR^8$ eine Abgangsgruppe darstellt, die durch $HPR^2_2$ ersetzt werden kann, und
R und $R^1$ wie in einem der Ansprüche 1 bis 5 definiert sind, mit $HPR^2_2$ umfaßt,
wobei $R^2$ wie in einem der Ansprüche 1 bis 3 definiert ist.

**14.** Verbindungen mit der Formel (V)

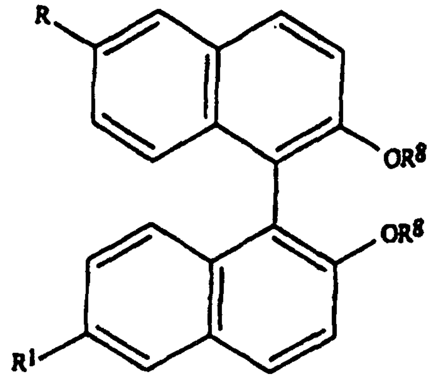

(V)

wobei

OR8 eine Abgangsgruppe darstellt, die durch $HPR_2^2$ ersetzt werden kann, und

R und $R^1$ wie in einem der Ansprüche 1 bis 5 definiert sind.

**15.** Verbindungen nach Anspruch 14, wobei $R^8$ $SO_2CF_3$ bedeutet.

**16.** Verbindungen mit Formel (III)

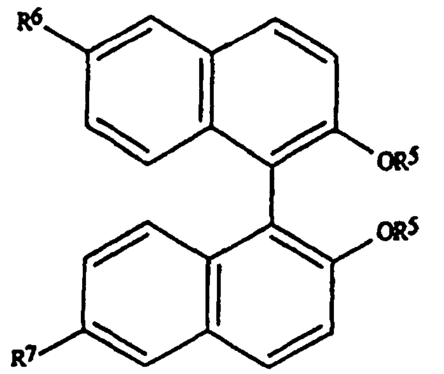

(III)

wobei

$R^5$ eine beliebige Alkylgruppe darstellt, die eine Substitution in Position 6 lenkt,

$R^6$ Cl, eine unverzweigte oder verzweigte aliphatische Acyl- oder nicht-Acylkette bedeutet, die wahlweise in einer Säurefunktion endet und wahlweise innerhalb der Kette oder am Kettenende eine oder mehrere aromatische Kohlenwasserstoff-, Ether-, Ester- oder Amidgruppen enthält, oder $R^6$ eine Phenylgruppe bedeutet, die wahlweise substituiert ist durch ein oder mehrere F, Cl, Br, $NO_2$, Aminogruppen, Naphtalengruppen, Anthracengruppen, Biphenylgruppen, $C_{1-6}$-Alkylgruppen, $CF_3$, CN, OH, O-$C_{1-6}$-Alkylgruppen, $CO_2H$, CHO, NHCO($C_{1-6}$-Alkyl)-Gruppen, $CO_2$($C_{1-6}$-Alkyl)-Gruppen, N($C_{1-6}$-Alkyl) CO-Gruppen, Benzylgruppen, zykli-

sche $C_{5-6}$-Ethergruppen oder ungesättigte $C_{2-4}$-Kohlenwasserstoffgruppen, oder $R^6$ eine heterozyklische Gruppe bedeutet, wie Thiophen, Furan, Pyridin, Pyrimidin, Chinolin, Benzofuran, Benzothiophen, Pyrrol, Imidazol, Isochinolin oder Indol, wobei die heterozyklischen Gruppen wahlweise durch eine oder mehrere Ether- oder $C_{1-6}$-Alkylgruppen substituiert sein können, und

$R^7$ $R^6$ oder H bedeutet,

mit der Bedingung, daß

a) $R^6$ nicht $-CO(CH_2)_3COOCH_3$, $-CO(CH_2)_3COOH$ oder $-(CH_2)_4COOH$ ist, wenn $R^5$ Methyl bedeutet und $R^7$ H bedeutet, und

b) $R^6$ kein $C_{1-30}$-Alkylrest, $C_{6-18}$-Arylrest oder $C_{7-30}$-Aralkylrest ist, wenn $R^7$ $R^6$ bedeutet.

**17.** Verbindungen nach Anspruch 16, wobei $R^5$ eine $C_{1-7}$-Alkyl- oder eine zyklische aliphatische $C_{3-7}$-Gruppe bedeutet.

**18.** Verbindungen nach Anspruch 17 oder Anspruch 18, wobei $R^5$ $(CH_2)_{0-6}CH_3$ bedeutet.

**19.** Verfahren für die Herstellung von Verbindungen nach einem der Ansprüche 1 bis 6, daß das Umsetzen der $OR^5$-Gruppen in einer Verbindung mit Formel (III) nach einem der Ansprüche 16 bis 18 in Abgangsgruppen umfaßt, die durch $HPR^2_2$ ersetzt werden können, Reaktion des Zielprodukts mit $HPR^2_2$, wobei $R^2$ wie in einem der Ansprüche 1 bis 3 definiert ist und, wenn notwendig, Durchführung von Synthesechemie, um die Gruppen $R^6$ und $R^7$ in Gruppen R bzw. $R^1$ umzusetzen.

**20.** Verfahren für die Herstellung von Verbindungen nach einem der Ansprüche 16 bis 18, das die Reaktion einer Verbindung mit Formel (IV)

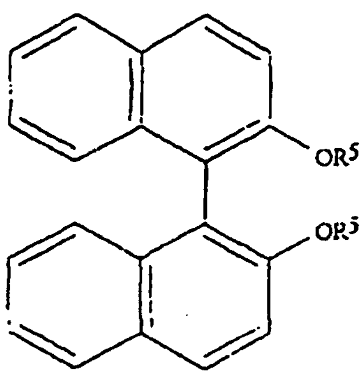

(IV)

wobei $R^5$ wie in einem der Ansprüche 16 bis 18 definiert ist,
mit einer Verbindung der Formel $R^6$Hal umfaßt,
wobei Hal Cl, Br oder I bedeutet und $R^6$ wie in einem der Ansprüche 16 bis 18 definiert ist, mit der Bedingung, daß $R^6$ nicht Cl bedeutet.

**21.** Verwendung von Komplexen nach einem der Ansprüche 7 bis 11 als Katalysatoren in asymmetrischen Reduktionsredaktionen.

**Revendications**

**1.** Composés de formule générale (I) :

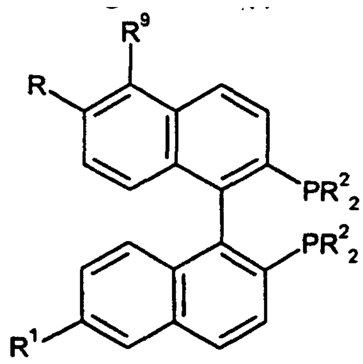

(I)

dans laquelle

R désigne un radical alkyle en $C_1$ à $C_6$, alcényle en $C_1$ à $C_6$, alcynyle en $C_1$ à $C_6$ ou phényle, où les groupes alkyle en $C_1$ à $C_6$ et phényle peuvent éventuellement être substitués par un ou plusieurs substituants qui peuvent comprendre F, Cl, Br, $NO_2$, amino, naphtalène, anthracène, biphényle, alkyle en $C_1$ à $C_6$, $CF_3$, CN, OH, O-alkyle en $C_1$ à $C_6$, $CO_2H$, CHO, NHCO(alkyle en $C_1$ à $C_6$), $CO_2$(alkyle en $C_1$ à $C_6$), N(alkyl en $C_1$ à $C_6$)CO, benzyle, les éthers cycliques en $C_5$ - $C_6$ ou les groupes hydrocarbonés insaturés en $C_2$ à $C_4$, et où le groupe alkyle en $C_1$ à $C_6$ peut éventuellement comprendre un ou plusieurs hétéroatomes ou groupes aryle intervenants dans la chaîne, ou bien R désigne CN, $CO_2NHR^3$, $(CH_2)_nOR^3$, $CO_2R^3$, benzyle, les groupes hétérocycliques tels que thiophène, furane, pyridine, pyrimidine, quinoline, benzofurane, benzothiophène, pyrrole, imidazole, isoquinoline ou indole, où les groupes hétérocycliques peuvent être éventuellement substitués par un ou plusieurs groupes éther ou alkyle en $C_1$ à $C_6$, ou Y-X-$R^4$ ;

$R^1$ désigne R ou H ;

$R^2$ désigne un radical phényle, phényle substitué par un ou plusieurs groupes alkyle en $C_1$ à $C_7$, groupes O-alkyle en $C_1$ à $C_6$ et/ou atomes d'halogène, ou bien $R^2$ désigne un groupe hydrocarboné aliphatique ;

$R^9$ désigne H ou, conjointement avec R, forme un cycle hydrocarboné à 5, 6 ou 7 chaînons, éventuellement substitué par un ou plusieurs groupes C=O, OH ou amine ;

Y désigne une chaîne aliphatique linéaire ou ramifiée, éventuellement incorporant un ou plusieurs groupes hydrocarbonés aromatiques ou liaisons éther dans la chaîne, ou un groupe hydrocarboné aromatique ;

x désigne $CH_2$, $CO_2$, O, CONH, NH, $CONR^2$, $NR^2$ ou une liaison de valence ;

$R^3$ désigne H ou un radical alkyle en $C_1$ à $C_{10}$, benzyle ou phényle ; et

$R^4$ désigne H, un radical alkyle en $C_1$ à $C_6$, un support insoluble, ou un groupe écarteur rattaché à un support insoluble ;

et tous les énantiomères, mélanges, y compris mélanges racémiques, et diastéréomères de tels composés.

**2.** Composés selon la revendication 1, dans lesquels

$R^2$ désigne le radical phényle ;

X désigne CONH ou $CO_2$ ;

Y désigne $(CH_2)_n$ où n vaut de 2 à 4 ; et

$R^1$ est identique à R.

**3.** Composés selon la revendication 1 ou la revendication 2, dans lesquels

$R^2$ désigne le radical phényle ;

$R^4$ désigne un support insoluble ou un groupe écarteur rattaché à un support insoluble ;

X désigne CONH ou $CO_2$ ; et

Y désigne $(CH_2)_n$ où n vaut de 2 à 4.

4. Composés selon l'une quelconque des revendications 1 à 3, répondant à l'une des formules suivantes:

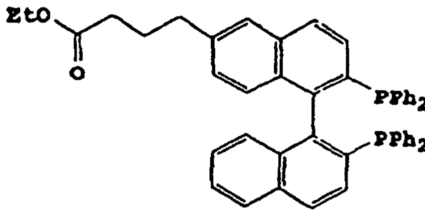

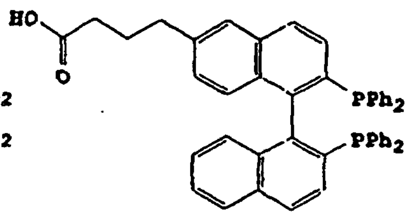

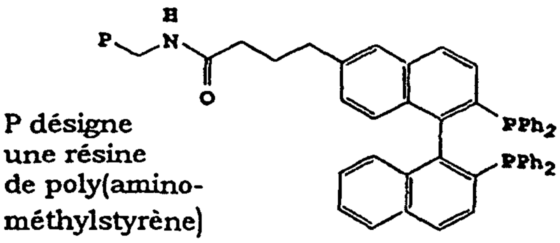

P désigne une résine de poly(amino-méthylstyrène)

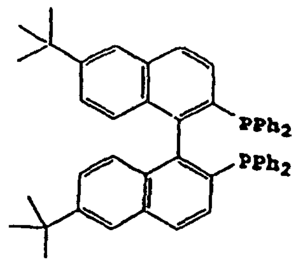

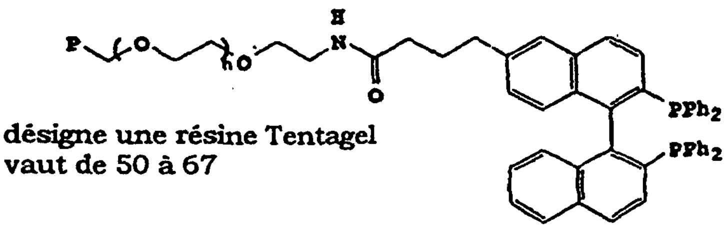

P désigne une résine Tentagel
n vaut de 50 à 67

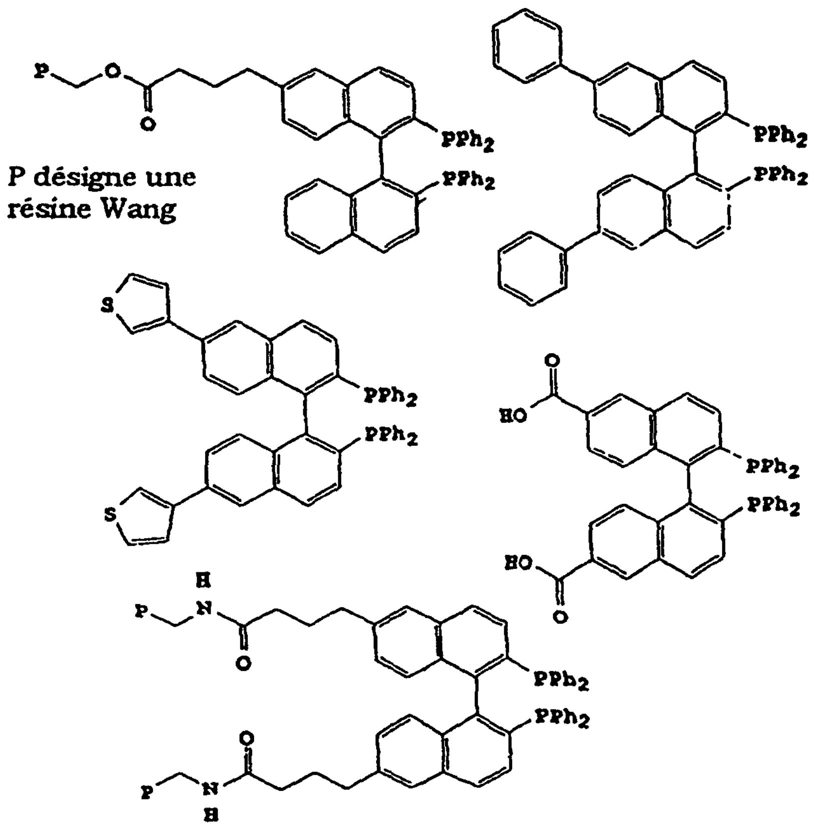

P désigne une résine Wang

5. Composés selon l'une quelconque des revendications 1 à 3, dans lesquels le support est un copolymère de polystyrène/ divinylbenzène (résine Merrifield), une résine de polystyrène, du polyamide, une résine de polystyrène aminométhylé, une résine Wang, une résine Tentagel aminométhylée, des composites de polyamide/terre de diatomées, un polyhipe, du coton ou du papier.

6. Composés selon l'une quelconque des revendications 1 à 5, qui sont constitués d'un énantiomère unique.

7. Complexes comprenant un composé de formule (I) selon l'une quelconque des revendications 1 à 6, complexé à un métal de transition.

8. Complexes selon la revendication 7, dans lesquels le métal de transition est le rhodium, le ruthénium, le palladium, l'iridium, le nickel, le cobalt ou le molybdène.

9. Complexes selon la revendication 7 ou la revendication 8, dans lesquels le métal de transition est le ruthénium, le rhodium ou le palladium.

10. Complexes selon l'une quelconque des revendications 7 à 9, de formule empirique $LRuBr_2$, où L désigne un composé de formule générale (I) selon l'une quelconque des revendications 1 à 6.

11. Complexes selon la revendication 10, dans lesquels L est un composé de formule (II) :

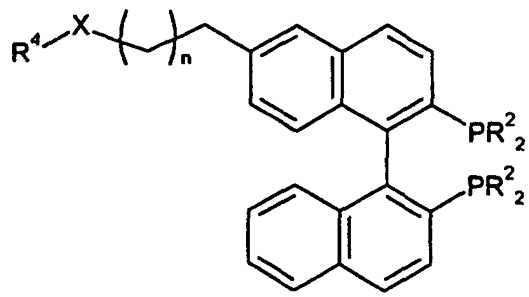

(II)

dans laquelle

X désigne $CO_2$, O, NH, CONH, $CH_2$ ou une liaison de valence ;
N vaut de 0 à 9 ; et

$R^2$ et $R^4$ sont tels que définis dans l'une quelconque des revendications 1 à 3.

**12.** Procédé pour la préparation de composés selon l'une quelconque des revendications 1 à 6 dans lesquels $R^1$ désigne Y-X-$R^4$ et $R^4$ désigne un support insoluble, qui comprend la liaison d'un composé de formule (I), dans lequel $R^4$ ne désigne pas un support insoluble, à un support insoluble.

**13.** Procédé pour la préparation de composés selon l'une quelconque des revendications 1 à 6, qui comprend la réaction d'un composé de formule (V) :

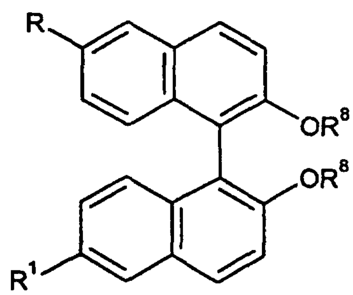

(V)

dans laquelle

$OR^8$ désigne un groupe partant qui peut être déplacé par $HPR^2_2$ ; et

R et $R^1$ sont tels que définis dans l'une quelconque des revendications 1 à 5 ;
avec $HPR^2_2$,
où $R^2$ est tel que défini dans l'une quelconque des revendications 1 à 3.

**14.** Composés de formule (V) :

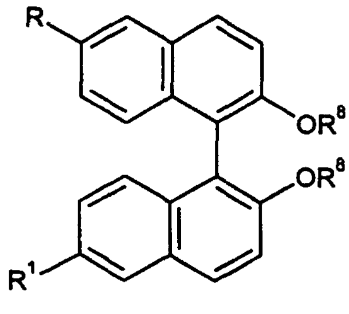

(V)

dans laquelle

$OR^8$ désigne un groupe partant qui peut être déplacé par $HPR^2_2$ ; et

R et $R^1$ sont tels que définis dans l'une quelconque des revendications 1 à 5.

**15.** Composés selon la revendication 14, dans lesquels $R^8$ désigne $SO_2CF_3$.

**16.** Composés de formule (III) :

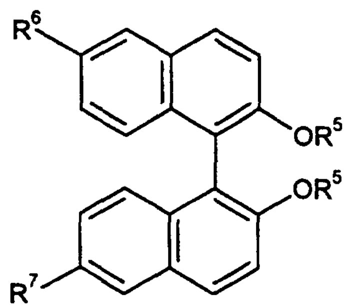

(III)

dans laquelle

$R^5$ désigne un groupe alkyle qui dirige une substitution en position 6;
$R^6$ désigne Cl, une chaîne aliphatique acyle ou non-acyle linéaire ou ramifiée, éventuellement se terminant en une fonctionnalité acide et éventuellement incorporant un ou plusieurs groupes hydrocarbonés aromatiques, éther, ester ou amide à l'intérieur de la chaîne ou en terminaison de chaîne ; ou bien $R^6$ désigne un groupe phényle, éventuellement substitué par un ou plusieurs parmi F, Cl, Br, $NO_2$, amino, naphtalène, anthracène, biphényle, alkyle en $C_1$ à $C_6$, $CF_3$, CN, OH, O-alkyle en $C_1$ à $C_6$, $CO_2H$, CHO, NHCO(alkyle en $C_1$ à $C_6$), $CO_2$(alkyle en $C_1$ à $C_6$), N(alkyl en $C_1$ à $C_6$)CO, benzyle, éthers cycliques en $C_5$ à $C_6$ ou groupes hydrocarbonés insaturés en $C_2$ à $C_4$, ou bien $R^6$ désigne un groupe hétérocyclique tel que thiophène, furane, pyridine, pyrimidine, quinoline, benzofurane, benzothiophène, pyrrole, imidazole, isoquinoline ou indole, où les groupes hétérocycliques peuvent être éventuellement substitués par un ou plusieurs groupes éther ou alkyle en $C_1$ à $C_6$ ; et
$R^7$ désigne $R^6$ ou H ;

avec les conditions suivantes :

a) quand $R^5$ désigne le radical méthyle et $R^7$ désigne H, alors $R^6$ ne désigne pas $-CO(CH_2)_3COOCH_3$, $-CO(CH_2)_3COOH$ ou $-(CH_2)_4COOH$ ; et
b) quand $R^7$ désigne $R^6$, alors $R^6$ ne désigne pas un radical alkyle en $C_1$ à $C_{30}$, aryle en $C_6$ à $C_{18}$ ou aralkyle

en $C_7$ à $C_{30}$.

**17.** Composés selon la revendication 16, dans lesquels $R^5$ désigne un radical alkyle en $C_1$ à $C_7$ ou un groupe aliphatique cyclique en $C_3$ à $C_7$.

**18.** Composés selon la revendication 16 ou la revendication 17, dans lesquels $R^5$ désigne $(CH_2)_{0-6}CH_3$.

**19.** Procédé pour la préparation de composés selon l'une quelconque des revendications 1 à 6, qui comprend la conversion des groupes $OR^5$ dans un composé de formule (III) selon l'une quelconque des revendications 16 à 18 en groupes partants qui peuvent être déplacés par $HPR^2_2$, à faire réagir le produit résultant avec $HPR^2_2$ où $R^2$ est tel que défini dans l'une quelconque des revendications 1 à 3, et, si nécessaire, à réaliser une chimie de synthèse pour convertir les groupes $R^6$ et $R^7$ en groupes R et $R^1$ respectivement.

**20.** Procédé pour la préparation de composés selon l'une quelconque des revendications 16 à 18, qui comprend la réaction d'un composé de formule (IV) :

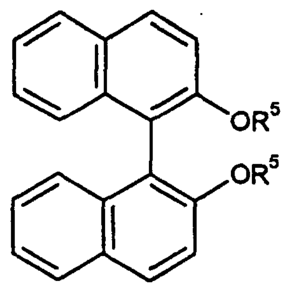

(IV)

dans laquelle $R^5$ est tel que défini dans l'une quelconque des revendications 16 à 18,

avec un composé de formule $R^6Hal$,
où Hal désigne Cl, Br ou I et $R^6$ est tel que défini dans l'une quelconque des revendications 16 à 18, à la condition que $R^6$ ne désigne pas Cl.

**21.** Utilisation de complexes selon l'une quelconque des revendications 7 à 11 comme catalyseurs dans des réactions de réduction asymétrique.